(19) Europäisches Patentamt / European Patent Office / Office européen des brevets

(11) **EP 3 509 483 B1**

(12) **FASCICULE DE BREVET EUROPEEN**

(45) Date de publication et mention de la délivrance du brevet:
**18.11.2020 Bulletin 2020/47**

(21) Numéro de dépôt: **17771820.2**

(22) Date de dépôt: **08.09.2017**

(51) Int Cl.:
*A61B 5/026* (2006.01)    *A61B 5/055* (2006.01)

(86) Numéro de dépôt international:
**PCT/FR2017/052387**

(87) Numéro de publication internationale:
**WO 2018/046864 (15.03.2018 Gazette 2018/11)**

(54) **SYSTEME ET PROCEDE POUR RECONSTRUIRE UN SIGNAL PHYSIOLOGIQUE D'UN SYSTEME DYNAMIQUE ARTERE/TISSU/VEINE D'UN ORGANE DANS UN ESPACE SURFACIQUE**

SYSTEM UND VERFAHREN ZUR REKONSTRUKTION EINES PHYSIOLOGISCHEN SIGNALS EINER DYNAMISCHEN ARTERIEN-/GEWEBE-/VENENSYSTEMS EINES ORGANS IN EINEM OBERFLÄCHENAREAL

SYSTEM AND METHOD FOR RECONSTRUCTING A PHYSIOLOGICAL SIGNAL OF AN ARTERY/TISSUE/VEIN DYNAMIC SYSTEM OF AN ORGAN IN A SURFACE SPACE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.09.2016 FR 1658426**

(43) Date de publication de la demande:
**17.07.2019 Bulletin 2019/29**

(73) Titulaires:
- **Olea Medical**
  **13600 La Ciotat (FR)**
- **Université d'Aix Marseille**
  **13007 Marseille (FR)**
- **Centre National de la Recherche Scientifique**
  **75016 Paris (FR)**

(72) Inventeurs:
- **THIEBAUT LONJARET, Lucie**
  **13002 Marseille (FR)**
- **BAKHOUS, Christine**
  **Montreal, QC H2X0A9 (CA)**
- **COULON, Olivier**
  **13360 Roquevaire (FR)**
- **TAKERKART, Sylvain**
  **13012 Marseille (FR)**

(74) Mandataire: **Med'inVent Consulting**
**Espace Mistral Bâtiment A**
**297, avenue du Mistral**
**13705 La Ciotat Cedex (FR)**

(56) Documents cités:
- **OPERTO G ET AL: "Projection of fMRI data onto the cortical surface using anatomically-informed convolution kernels", NEUROIMAGE, ELSEVIER, AMSTERDAM, NL, vol. 39, no. 1, 1 janvier 2008 (2008-01-01), pages 127-135, XP022931612, ISSN: 1053-8119, DOI: 10.1016/J.NEUROIMAGE.2007.08.039 [extrait le 2008-01-01]**
- **GROVA C ET AL: "Anatomically informed interpolation of fMRI data on the cortical surface", NEUROIMAGE, ELSEVIER, AMSTERDAM, NL, vol. 31, no. 4, 15 juillet 2006 (2006-07-15), pages 1475-1486, XP024906352, ISSN: 1053-8119, DOI: 10.1016/J.NEUROIMAGE.2006.02.049 [extrait le 2006-07-15]**
- **BAILLET S ET AL: "A Bayesian approach to introducing anatomo-functional priors in the EEG/MEG inverse problem", IEEE TRANSACTIONS ON BIOMEDICAL ENGINEERING IEEE USA, vol. 44, no. 5, mai 1997 (1997-05), pages 374-385, XP002772122, ISSN: 0018-9294**
- **LONJARET L T ET AL: "ISA - an inverse surface-based approach for cortical fMRI data projection", 2017 IEEE 14TH INTERNATIONAL SYMPOSIUM ON BIOMEDICAL IMAGING (ISBI) IEEE PISCATAWAY, NJ, USA, avril 2017 (2017-04), pages 1104-1107, XP002772123, ISBN: 978-1-5090-1172-8**

**(Cont. page suivante)**

- Peter Lincoln: "Surface Projection Method for Visualizing Volumetric Data", , juin 2006 (2006-06), XP002772124, Extrait de l'Internet: URL:http://sigpubs.biostr.washington.edu/archive/00000193/01/LincolnFinalReport.pdf [extrait le 2017-07-14]
- RICHARD N. HENSON,GUILLAUME FLANDIN,KARL J. FRISTON,JÉRÉMIE MATTOUT: "A Parametric Empirical Bayesian framework for fMRI-constrained MEG/EEG source reconstruction", HUMAN BRAIN MAPPING, vol. 31, no. 10, octobre 2010 (2010-10), pages 1512-1531, XP002772125, DOI: 10.1002/hbm.20956

**EP 3 509 483 B1**

**Description**

L'invention concerne un système et un procédé pour reconstruire un signal physiologique d'un système dynamique artère/tissu/veine d'un organe dans un espace surfacique. Un tel procédé permet notamment de générer une image sous la forme d'une carte d'activité fonctionnelle. OPERTO G ET AL: "Projection of fMRI data onto the cortical surface using anatomically-informed convolution kernels",NEUROIMAGE, ELSEVIER, AMSTERDAM, NL, vol. 39, no. 1, 1 janvier 2008 (2008-01-01), pages 127-135, ISSN: 1053-8119, DOI: 10.1016/J.NEUROIMAGE.2007.08.039 divulgue un système et un procédé connu. L'invention se distingue notamment des procédés connus par sa grande précision et par sa robustesse au bruit.

Selon un exemple de réalisation préféré mais non limitatif, l'invention sera décrite dans son application avec le cerveau. Toutefois, l'invention ne saurait être limitée à ce seul organe et pourrait être appliquée par exemple au sein ou au rein.

L'invention s'appuie notamment sur des techniques d'Imagerie par Résonance Magnétique (également connue sous l'abréviation « IRM » ou encore sous la terminologie anglo-saxonne « *Magnetic Resonance Imaging* » - MRI), plus particulièrement fonctionnelle (également connue sous l'abréviation « IRMf » ou encore sous la terminologie anglo-saxonne « *Functional Magnetic Resonance Imaging* » - fMRI). Ces techniques permettent d'obtenir rapidement des informations précieuses sur les organes des êtres humains ou des animaux. Ces informations sont particulièrement cruciales pour un praticien cherchant à établir un diagnostic et à prendre une décision thérapeutique dans le traitement de pathologies. Bien qu'employée préférentiellement en lien avec l'Imagerie par Résonance Magnétique fonctionnelle, l'invention ne saurait toutefois être limitée à ce seul type d'imagerie ou ce seulprotocole d'acquisition. A ce titre, l'invention pourrait avantageusement être employée dans tout autre protocole visant à étudier tout signal fonctionnel, éventuellement cortical, tel que, à titre d'exemple non limitatif, la tomographie par émission de positon (également connue sous l'abréviation « TEP » et sous la terminologie anglo-saxonne « positron emission tomography - PET »).

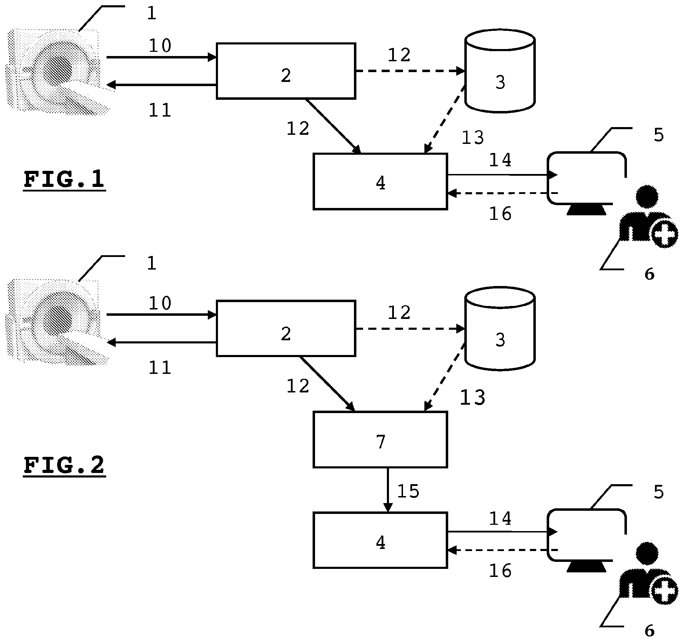

Pour mettre en œuvre de telles techniques, un appareil d'imagerie 1 par Résonance Magnétique Nucléaire, tel qu'illustré à titre d'exemple non limitatif par les figures 1 et 2, est généralement utilisé. Celui-ci peut délivrer une pluralité de séquences d'images numériques 12 d'une ou plusieurs parties du corps d'un patient, à titre d'exemples non limitatifs, le cerveau, le cœur ou encore les poumons. Ledit appareil applique pour cela une combinaison d'ondes électromagnétiques à haute fréquence sur la partie du corps considérée et mesure le signal réémis par certains atomes, tels qu'à titre d'exemple non limitatif, l'hydrogène pour l'imagerie par Résonance Magnétique Nucléaire. L'appareil permet ainsi de déterminer les propriétés magnétiques, et par voie de conséquence, la composition chimique des tissus biologiques et donc leur nature en chaque volume élémentaire, que l'on nomme communément un voxel, du volume imagé. L'appareil 1 d'imagerie par Résonance Magnétique Nucléaire est commandé à l'aide d'une console 2. Un utilisateur peut ainsi choisir des paramètres 11 pour piloter l'appareil 1. A partir d'informations 10 produites par ledit appareil 1, on obtient une pluralité de séquences d'images numériques 12 d'une partie d'un corps d'un humain ou d'un animal.

Les séquences d'images 12 peuvent optionnellement être stockées au sein d'un serveur 3 et constituer un dossier médical 13 d'un patient. Un tel dossier 13 peut comprendre des images de différents types, telles que des images fonctionnelles, mettant en évidence l'activité des tissus, ou des images anatomiques, reflétant les propriétés des tissus. Les séquences d'images 12 sont analysées au moyen d'une unité de traitement 4 dédiée. Ladite unité de traitement 4 comporte des moyens pour communiquer avec le monde extérieur pour recueillir les images. Lesdits moyens pour communiquer permettent en outre à l'unité de traitement 4 de délivrer in fine, par l'intermédiaire de moyens de restitution 5 proposant un rendu graphique, sonore ou autre, à un utilisateur 6 du système d'analyse, notamment un praticien ou un chercheur, une estimation d'un ou plusieurs signaux physiologiques, éventuellement formatée sous la forme d'un contenu, à partir des images 12 obtenues par Imagerie par Résonance Magnétique, au moyen d'une interface homme-machine adaptée. Dans tout le document, on entend par « moyens de restitution », tout dispositif, employé seul ou en combinaison, permettant de restituer une représentation, par exemple graphique, sonore ou autre, d'un signal physiologique reconstruit, à l'utilisateur 6 d'un système d'analyse d'imagerie par Résonance Magnétique. De tels moyens de restitution 5 peuvent consister, de manière non exhaustive, en un ou plusieurs écrans, haut-parleurs ou autres interfaces homme-machine. Ledit utilisateur 6, éventuellement un praticien, du système d'analyse peut ainsi confirmer ou infirmer un diagnostic, décider d'une action thérapeutique qu'il jugera adéquate, approfondir des travaux de recherche... Optionnellement, cet utilisateur 6 peut paramétrer le fonctionnement de l'unité de traitement 4 ou des moyens de restitution 5, au moyen de paramètres 16. Par exemple, il peut ainsi définir des seuils d'affichage ou choisir les signaux reconstruits pour lesquels il souhaite disposer d'une représentation, par exemple graphique. Il existe une variante, décrite en liaison avec la figure 2, pour laquelle un système d'imagerie, tel que décrit précédemment, comporte en outre une unité de prétraitement 7 pour analyser les séquences d'images, en déduire des signaux expérimentaux 15 et délivrer ces derniers à l'unité de traitement 4 qui est ainsi déchargée de cette tâche. Par ailleurs, pour réaliser une reconstruction de signaux physiologiques, l'unité de traitement 4 comporte généralement des moyens de traitement, tels qu'un ou plusieurs calculateurs ou processeurs, pour mettre en œuvre un procédé de reconstruction sous la forme d'un programme préalablement chargé dans des moyens de mémorisation coopérant avec lesdits moyens de traitement.

Ainsi, l'acquisition d'une ou plusieurs données expérimentales, avantageusement un ou plusieurs signaux expérimentaux, par Imagerie de Résonance Magnétique, peut être effectuée en échantillonnant régulièrement un volume parallélépipédique selon un plan de coupe donné. Les images en deux dimensions obtenues sont constituées de pixels dotés d'une épaisseur, correspondant à l'épaisseur de la coupe et appelés voxels. Une telle technique d'imagerie permet ainsi d'acquérir des images aussi bien anatomiques, pour permettre par exemple de refléter les propriétés des tissus, que fonctionnelles, pour mettre en évidence par exemple l'activité des tissus.

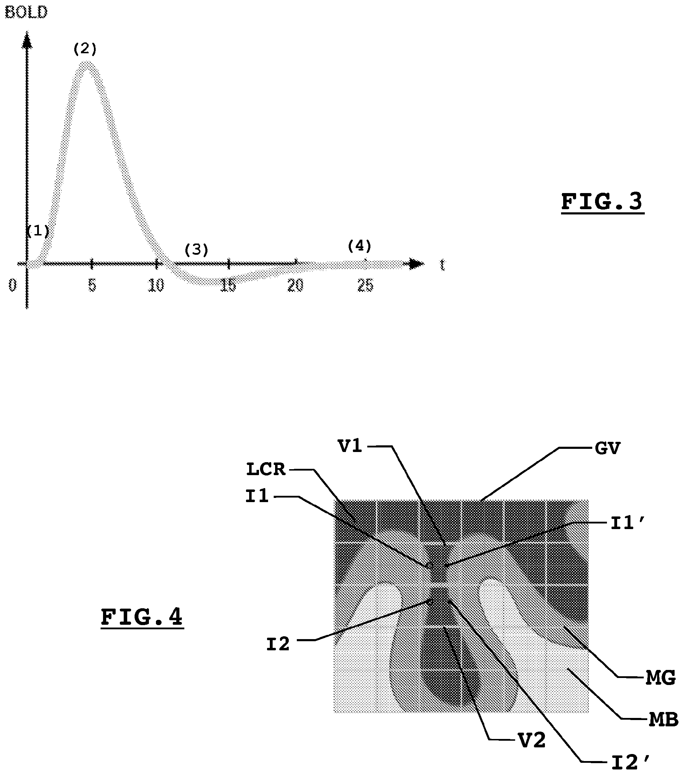

En IRMf, la mesure de l'activité neuronale est indirecte. En effet, aucun appareil et/ou aucune technique ne sont adaptés et/ou agencés pour garantir une telle mesure. Toutefois, des études ont démontré que l'activité cérébrale, plus particulièrement neuronale, avait un impact direct sur le flux sanguin et sa composition. Aussi, des méthodes employant l'IRMf peuvent comporter des étapes pour enregistrer les variations hémodynamiques cérébrales locales, à savoir au sein de la matière grise, lorsque celle-ci est en activité, ladite activation se répercutant sur la valeur prise par le voxel représentant cette partie de matière grise. Ainsi, ce sont les changements qu'une telle activité neuronale entraîne au niveau sanguin qui peuvent être finalement estimés. Le signal BOLD (« *dépendant du niveau d'oxygène dans le sang* » - « *Blood Oxygenation Level Dépendent* » selon une terminologie anglo-saxonne), c'est-à-dire un signal reflétant les variations locales et transitoires de la concentration en hémoglobine oxygénée dans le sang en fonction de l'activité neuronale du cerveau, peut alors être étudié. L'étude du signal BOLD, en conséquence, repose sur l'analyse du ratio d'hémoglobine oxygénée (également connue sous la terminologie « *oxyhémoglobine* »), possédant des propriétés diamagnétiques par rapport à l'hémoglobine désoxygénée (également connue sous la terminologie « *désoxyhémoglobine* »), possédant quant à elle des propriétés paramagnétiques, dans le sang. De par ses propriétés paramagnétiques, l'hémoglobine désoxygénée entraîne une diminution du signal IRM. En réalité, lorsqu'une zone neuronale est activée, la demande locale en énergie, c'est-à-dire la demande d'apport en nutriments et oxygène, augmente. Pour répondre à cette augmentation de la demande, le flux sanguin augmente alors dans une telle zone neuronale, de manière beaucoup plus importante en comparaison, que le besoin en oxygène. Ainsi, une diminution de la concentration en désoxyhémoglobine, et par voie de conséquence une augmentation du signal BOLD, est observée dans la zone activée. Les évolutions inhérentes à une telle activation neuronale peuvent être décrites par une fonction, la fonction de réponse hémodynamique (« *HRF - Hemodynamic Response Function* » selon une terminologie anglo-saxonne). Une telle fonction de réponse hémodynamique permet d'observer les variations du signal BOLD en fonction du temps. La figure 3 présente ladite fonction de réponse hémodynamique. Une telle fonction se décompose principalement en quatre phases. Selon la figure 3, t=0 correspond à l'instant auquel le neurone s'active : le signal BOLD montre tout d'abord une légère décroissance (représentée sur la courbe par la référence (1)), avant d'augmenter franchement jusqu'à atteindre un pic maximal (représenté sur la courbe par la référence (2)), autour de cinq à six secondes après l'activation du neurone, suivi d'une nouvelle décroissance (représentée sur la courbe par la référence (3)), jusque sous la ligne de base autour de dix à quinze secondes, pour ensuite revenir à la ligne de base autour de vingt-cinq secondes (situation représentée sur la courbe par la référence (4)).

Par ailleurs, le signal BOLD correspondant à un ratio, il n'existe pas d'échelle absolue pour la mesure dudit signal BOLD. En outre, l'amplitude dudit signal BOLD dépend de nombreux facteurs tels que, non limitativement, les caractéristiques inhérentes à l'appareil et/ou au système d'imagerie fonctionnelle, les paramètres d'acquisition employés ou encore le type de tissus traversés, notamment la concentration locale en eau desdits tissus. Afin de réduire les incertitudes introduites par tous ces facteurs lors de l'observation du signal BOLD, le pourcentage de changement de signal (également connu sous l'abréviation « PSC »), correspondant au ratio de la différence entre la valeur du signal et la valeur de ligne de base sur la valeur de la ligne de base, est généralement utilisé pour caractériser le signal BOLD. En IRMf, les valeurs de PSC généralement rencontrées se situent entre 0.1% et 5%, rendant les variations particulièrement difficiles à mettre en évidence à l'échelle d'un individu. Effectivement, sans une augmentation du rapport signal sur bruit, ledit bruit tend à masquer les faibles fluctuations dudit signal BOLD. Par ailleurs, les résolutions spatiale et temporelle sont relativement limitées en IRMf lorsque lesdits volumes de données expérimentales sont acquis. En effet, de tels volumes de données expérimentales présentent alors une précision approximative, entrainant alors une perte d'informations contraignante.

Par conséquent, après leur acquisition, les signaux expérimentaux, sous la forme de volumes de données, sont généralement post-traités et analysés dans leur espace d'acquisition par le biais de techniques basées sur les voxels. Toutefois, de telles techniques basées sur les voxels présentent un certain nombre d'inconvénients. Le principal inconvénient de ces techniques est de n'accorder que trop peu d'importance et d'intérêt aux caractéristiques structurelles et/ou anatomiques du cerveau, plus généralement de l'organe à étudier. De ce fait, les informations obtenues ne sont pas toujours fiables, puisque de telles informations relatives, par exemple, aux signaux corticaux BOLD provenant de côtés opposés d'un sillon, peuvent éventuellement être « mélangées ». En effet, le faible rapport « contraste du signal BOLD sur bruit » observé en IRMf impose l'emploi dans ces techniques d'une étape de débruitage ou de rehaussement du signal d'intérêt. La méthode la plus commune pour réaliser une telle étape de débruitage est une étape de filtrage des données expérimentales acquises au moyen d'un filtre passe-bas, aboutissant à la production de données moyennées des données contenues dans les voxels sur leur voisinage direct. Cependant, le voisinage dans la « grille » des voxels ne correspond pas toujours au voisinage réel dans la structure très convoluée du cortex. La figure 4 présente un exemple d'une grille de voxels représentant un sillon d'une surface corticale d'un cerveau humain. Conformément à la figure 4, une telle grille de voxels GV possède trente voxels, représentés sur la figure 4 par trente carrés de taille identique. Est représentée sur ladite grille des voxels GV une vue en coupe d'un sillon du cerveau. Selon la figure 4, un sillon est une structure géométrique désignant les plis concaves du cerveau, à proximité de laquelle se trouvent trois types de tissus : la matière blanche MB, la matière grise MG et le liquide céphalo-rachidien LCR. Toujours sur la grille de voxels GV, deux des voxels V1 et V2 confondent respectivement les informations I1, I1' et I2, I2' provenant de part et d'autre du sillon. Dans le domaine géodésique, c'est-à-dire le long de la surface, lesdites informations I1, I1' et I2, I2' sont pourtant distinctes. En géométrie, le chemin le plus court, ou l'un des plus courts chemins s'il en existe plusieurs, entre deux points d'un espace pourvu d'une métrique ou distance est une géodésique. Ainsi, selon la figure 4, le chemin le plus court pour aller respectivement des informations I1, I2 aux informations I1', I2' passe par le fond du sillon. Par opposition, dans le domaine des voxels, les informations I1, I1' et I2, I2' se retrouvent respectivement dans les mêmes voxels V1, V2.

Les méthodes ou approches basées sur la surface corticale (également connues sous l'abréviation et la terminologie anglo-saxonne « SBM - *Surface Based Method* ») peuvent résoudre ce problème en étudiant les signaux corticaux dans leur espace surfacique d'origine, en l'espèce la surface corticale, afin de toujours prendre en compte la géométrie de cet espace d'origine. Étant données l'organisation anatomique du cortex en unités fonctionnelles (également connues sous le terme de « colonnes corticales ») perpendiculaires à la surface corticale, l'organisation microvasculaire cérébrale en vaisseaux sanguins suivant globalement lesdites colonnes, et la résolution d'acquisition (environ de trois millimètres) proche de l'épaisseur du cortex, il est effectivement possible d'assimiler, dans le cadre de signaux BOLD, le cortex à une surface. Parmi l'ensemble des approches SBM connues permettant cette assimilation, deux démarches et types de méthodes se distinguent : celles, dites géométriques, et celles, plus évoluées, dites anatomiquement informées.

Les méthodes géométriques, conceptuellement très simples, se basent généralement sur des procédés d'interpolation, c'est-à-dire des opérations mathématiques permettant de construire une courbe à partir d'un nombre fini de points, tels que par exemple, l'interpolation au plus proche voisin, l'interpolation trilinéaire, ou encore une convolution avec un filtre ellipsoïdal, ou tout simplement sur l'attribution, à chaque nœud d'un maillage surfacique, de la valeur du voxel contenant le nœud. Chacune de ces techniques, malgré l'avantage que leur confère leur simplicité, comporte un certain nombre d'inconvénients, principalement liés au rapport étroit entre les caractéristiques propres des images et la méthode de projection surfacique employée. Ainsi, bien qu'une approche géométrique puisse, par exemple, préserver les relations d'adjacence spatiale, elle peut éventuellement entraîner la perte d'informations pertinentes, telles que la taille des activations neuronales. Quelle que soit l'approche géométrique employée, cette dernière n'observe généralement pas de différences dans les informations relatives aux signaux issus du liquide céphalo-rachidien (également connu sous l'abréviation LCR et sous la terminologie anglo-saxonne « *cerebrospinal fluid - CSF* »), de la matière blanche (également connue sous l'abréviation MB et sous la terminologie anglo-saxonne *« white matter »*), et/ou de la matière grise (également connue sous l'abréviation MG et sous la terminologie anglo-saxonne « *grey matter* »), ce qui s'avère être une aberration relativement aux signaux corticaux, puisque les activations neuronales sont localisées à la matière grise MG uniquemement. De plus, de telles méthodes géométriques souffrent d'un manque de robustesse face à des erreurs de segmentation et/ou des erreurs de recalage anatomo-fonctionnel.

Les méthodes anatomiquement informées, basées sur le principe des approches surfaciques, tentent quant à elles de représenter les signaux fonctionnels dans leur espace d'origine afin de restituer leurs caractéristiques initiales en tenant compte notamment des spécificités anatomiques du cortex. Dans ce but, de telles méthodes anatomiquement informées introduisent des informations supplémentaires liées a minima à l'anatomie, dans certains cas à la physiologie de l'organe étudié ou aux caractéristiques inhérentes aux modalités d'imagerie, notamment d'acquisition, employées.

Différents chercheurs, parmi lesquels Messieurs Kiebel, Grova, Warnking ou encore Operto se sont prêtés à l'exercice, ont développé des méthodes anatomiquement informées, en employant notamment des méthodes de reprojection d'un signal physiologique dans un espace surfacique décrivant la géométrie du domaine sur lequel est défini ledit signal physiologique à partir d'une ou plusieurs données expérimentales acquises par un système d'analyse d'imagerie fonctionnelle, en tenant compte d'informations a priori relatives à l'anatomie, à la physiologie de l'organe étudié ou aux caractéristiques inhérentes aux modalités d'imagerie, notamment d'acquisition, employée. Généralement, on définit le terme « projection » comme le passage d'un premier espace ou référentiel à un deuxième espace ou référentiel, au moyen de méthodes mathématiques adaptées, par exemple, la méthode des moindres carrés. Ainsi, Au sens de l'invention et dans tout le document, on entend par « re-projection » tout changement d'espace ou de référentiel effectué dans le but de se replacer dans l'espace ou dans le référentiel surfacique décrivant la géométrie de l'organe dont le signal ou les signaux physiologiques sont issus, en l'espèce, dans le cadre de l'exemple d'application préférée en lien avec le cerveau, la surface corticale.

Les méthodes employées actuellement présentent encore de nombreux inconvénients, entraînant bien souvent une perte d'information conséquente et potentiellement la restitution d'un signal physiologique incomplet voire peu pertinent. En effet, les méthodes se doivent de considérer la différence entre la résolution des images acquises par Imagerie Résonance Magnétique fonctionnelle sous la forme de voxels et la dimension des colonnes corticales constituant principalement la surface corticale et contenant la ou les informations relatives au signal physiologique : tandis qu'un voxel a généralement des dimensions de l'ordre de quelques millimètres, plus particulièrement deux ou trois millimètres, une colonne corticale possède des dimensions de l'ordre du dixième de millimètres. Finalement, un voxel peut alors contenir simultanément la ou les informations de plusieurs colonnes. En outre, les données expérimentales, également connues sous le terme de « volumes fonctionnels », acquises par Imagerie par Résonance Magnétique fonctionnelle, correspondent généralement à des images en trois dimensions. Comme précisé précédemment, à chaque donnée expérimentale, sous la forme d'un voxel, est associée une unique valeur de signal expérimental. Cependant, un voxel peut être situé à la frontière entre deux tissus et le signal associé à une telle position de frontières reflète alors les phénomènes de Résonance Magnétique de deux entités aux propriétés tissulaires différentes. Aussi, un voxel peut potentiellement contenir des signaux ou informations issu(e)s respectivement de tissus différents et les mélanger sans aucune discrimination.

Comme précisé précédemment, une technique employable pour pallier à cet inconvénient peut consister en la projection des données expérimentales sur la surface corticale par le biais de méthodes connues, comme les approches par interpolation. Cependant, de telles méthodes souffrent d'un manque général de robustesse au bruit et ne tiennent généralement pas compte de la dimension temporelle. Or, comme d'ores et déjà énoncé au préalable, les données acquises par Imagerie par Résonance Magnétique fonctionnelle, et in fine le signal physiologique d'intérêt, sont bien souvent « polluées » par un niveau de bruit important. Dans une région d'intérêt, la présence ou l'absence d'activité fonctionnelle, par exemple neuronale, est détectée de par la dimension temporelle du signal. Pourtant, le traitement d'une telle dimension temporelle n'est réalisé qu'une fois la méthode de projection surfacique mise en œuvre et effectuée et le signal physiologique estimé, lors de la mise en œuvre d'un deuxième procédé consécutif visant à la détection d'activations fonctionnelles. Or, le bruit entachant la dimension temporelle résulte d'un processus initial et inhérent au processus d'acquisition des données expérimentales par le système. Ainsi, lors de la re-projection des données expérimentales et/ou du signal physiologique, les erreurs et biais relatifs au bruit sont également propagés au cours du procédé. Aussi, les méthodes actuelles n'offrent que des solutions partiellement efficaces. En effet, les informations relatives au signal physiologique re-projeté dans l'espace surfacique manquent ainsi de pertinence, puisque les méthodes actuelles ne traitent pas ou que trop peu la dimension temporelle des données expérimentales.

L'invention permet de répondre à tout ou partie des inconvénients soulevés par les solutions connues.

Parmi les nombreux avantages apportés par l'invention, nous pouvons mentionner que celle-ci permet :

- de proposer un procédé permettant la reconstruction d'un signal physiologique, indépendamment des caractéristiques de la zone active de l'organe étudié ;
- d'obtenir de meilleurs résultats en améliorant considérablement la robustesse au bruit, aussi bien spatialement que temporellement, permettant ainsi d'accroître la qualité du signal physiologique reconstruit et finalement la détection d'activations fonctionnelles ;
- d'accroître la robustesse des informations produites, malgré les éventuelles erreurs de recalage entre les données fonctionnelles et anatomiques, les erreurs de distorsion non-corrigées et/ou les erreurs de segmentation du volume anatomique.

A cette fin, il est notamment prévu un procédé pour reconstruire un signal physiologique d'un système dynamique artère/tissu/veine d'un organe dans un espace surfacique, ledit procédé étant mis en œuvre par des moyens de traitement d'une unité de traitement d'un système d'analyse d'imagerie fonctionnelle, et comportant une étape pour reconstruire ledit signal physiologique à partir d'une donnée expérimentale d'une région d'intérêt comportant un volume élémentaire - dit voxel - dudit organe et d'un maillage surfacique décrivant ledit espace surfacique. Selon l'invention, l'étape pour reconstruire ledit signal physiologique d'un tel procédé consiste à évaluer suivant une méthode de résolution d'un problème inverse une distribution marginale a posteriori pour ledit signal physiologique en un nœud dudit maillag e par :

- l'assignation d'une distribution de probabilité directe de la donnée expérimentale dans ledit espace surfacique sachant les paramètres intervenant dans le problème de reconstruction du signal physiologique du système dynamique artère/tissu/veine pour le voxel considéré ;
- l'assignation conjointe d'une distribution de probabilité spatiale a priori dudit signal physiologique par l'introduction d'une information a priori relative à une caractéristique de la donnée expérimentale et/ou d'une information a priori relative à une propriété du système dynamique artère/tissu/veine ;
- l'assignation conjointe d'une distribution de probabilité temporelle a priori dudit signal physiologique par l'introduction d'une information a priori relative à la réponse impulsionnelle dudit système dynamique artère/tissu/veine.

L'invention prévoit en outre, un procédé pour reconstruire un signal physiologique d'un système dynamique artère/tissu/veine d'un organe dans un espace surfacique, ledit procédé étant mis en œuvre par des moyens de traitement d'une unité de traitement d'un système d'analyse d'imagerie fonctionnelle, et comportant une étape pour reconstruire le signal physiologique à partir d'une donnée expérimentale d'une région d'intérêt comportant un volume élémentaire - dit voxel - et d'un maillage surfacique décrivant ledit espace surfacique. Selon l'invention, et tout comme précédemment, l'étape pour reconstruire ledit signal physiologique d'un tel procédé consiste à évaluer suivant une méthode de résolution d'un problème inverse une fonction de coût pour ledit signal physiologique en un nœud dudit maillage par :

- l'assignation d'un opérateur du modèle direct établissant le lien entre la donnée expérimentale dans le volume élémentaire et ledit signal physiologique dans ledit espace surfacique sachant les paramètres intervenant dans le problème de la reconstruction du signal physiologique du système dynamique artère/tissu/veine pour le voxel considéré ;
- l'assignation conjointe d'un opérateur de régularisation spatiale par l'introduction d'une information a priori relative à une caractéristique de la donnée expérimentale et/ou d'une information a priori relative à une propriété du système dynamique artère/tissu/veine ;
- l'assignation conjointe d'un opérateur de régularisation temporelle par l'introduction d'une information a priori relative à la réponse impulsionnelle dudit système dynamique artère/tissu/veine.

Pour permettre des diagnostics rapides et particulièrement efficaces, ainsi que des examens succincts, un procédé pour reconstruire un signal physiologique conforme à l'invention peut en outre comporter une étape pour produire ladite donnée expérimentale à partir d'une acquisition d'un signal par imagerie fonctionnelle.

Avantageusement, lorsque le système d'analyse d'imagerie fonctionnelle comporte des moyens de restitution du signal physiologique reconstruit à un utilisateur dudit système, lesdits moyens de restitution coopérant avec l'unité de traitement, un procédé selon l'invention peut comporter une étape subséquente pour déclencher une restitution du signal physiologique reconstruit selon un format approprié.

Pour améliorer la qualité des signaux expérimentaux obtenus et acquis par imagerie fonctionnelle et finalement la qualité des résultats obtenus, un procédé selon l'invention peut comporter en outre une étape préalable de prétraitement de la donnée expérimentale et/ou du maillage surfacique, ladite étape étant agencée pour corriger et/ou recaler respectivement la donnée expérimentale et/ou le maillage surfacique.

Avantageusement, lorsque le système d'analyse d'imagerie fonctionnelle comporte des moyens de restitution à un utilisateur dudit système, lesdits moyens de restitution coopérant avec l'unité de traitement, un procédé conforme à l'invention peut comporter en outre une étape subséquente pour déclencher la restitution du signal physiologique reconstruit en un ou plusieurs nœuds du maillage pour chaque voxel de la région d'intérêt et générer une image sous la forme d'une carte d'activité fonctionnelle.

Selon un deuxième objet, l'invention concerne une unité de traitement comportant des moyens pour communiquer avec le monde extérieur et des moyens de traitement, coopérant avec des moyens de mémorisation. De manière avantageuse, les moyens pour communiquer sont agencés pour recevoir du monde extérieur une donnée expérimentale et les moyens de mémorisation comportent des instructions exécutables ou interprétables par les moyens de traitement dont l'interprétation ou l'exécution desdites instructions par lesdits moyens de traitement provoque la mise en œuvre d'un procédé conforme au premier ou deuxième objet de l'invention.

Pour aider un praticien cherchant à établir un diagnostic, les moyens pour communiquer d'une unité de traitement conforme à l'invention peuvent délivrer un signal physiologique reconstruit selon un format approprié à des moyens de restitution aptes à la restituer à un utilisateur.

Selon un troisième objet, l'invention concerne un système d'analyse d'imagerie fonctionnelle comportant une unité de traitement conforme à l'invention et des moyens de restitution aptes à restituer à un utilisateur un signal physiologique selon un procédé conforme au premier objet de l'invention et mis en œuvre par ladite unité de traitement.

Enfin, selon un quatrième objet, l'invention concerne un produit programme d'ordinateur comportant une ou plusieurs instructions interprétables ou exécutables par les moyens de traitement d'une unité de traitement conforme à l'invention. Ladite unité de traitement comporte en outre des moyens de mémorisation ou coopérant avec de tels moyens de mémorisation, ledit programme étant chargeable dans lesdits moyens de mémorisation. Lesdites instructions par lesdits moyens de traitement sont telles que leur interprétation ou exécution provoque la mise en œuvre d'un procédé conforme au premier ou deuxième objet de l'invention.

D'autres caractéristiques et avantages apparaîtront plus clairement à la lecture de la description qui suit et à l'examen des figures qui l'accompagnent parmi lesquelles :

- les figures 1 et 2, précédemment décrites, présentent deux variantes de réalisation d'un système d'analyse d'imagerie médicale, éventuellement par Résonance Magnétique ;
- la figure 3, précédemment décrite, présente un exemple d'une fonction de réponse hémodynamique ;
- la figure 4, précédemment décrite, présente un exemple d'une grille partielle de voxels représentant un sillon d'une surface corticale d'un cerveau humain ;
- la figure 5 décrit schématiquement un organigramme simplifié d'un procédé conforme à l'invention ;
- les figures 6A, 6B et 6C présentent trois exemples de textures statiques, respectivement générée et restituée selon un procédé conforme à l'invention, correspondant au signal original ou témoin, générée et restituée selon un procédé conforme à l'Etat de la technique ;
- la figure 7 présente un ensemble de quatre exemples de décours temporels d'un signal physiologique en un même nœud, respectivement un signal physiologique original ou témoin, un signal physiologique bruité d'un voxel contenant ledit nœud, un signal physiologique re-projeté par un procédé conforme à l'invention et un signal physiologique reconstruit par un procédé selon l'Etat de la Technique ;
- les figures 8A, 8B et 8C présentent trois exemples de cartes d'activité fonctionnelle, respectivement générée et restituée à partir d'un signal physiologique respectivement reconstruit selon un procédé conforme à l'invention, correspondant au signal original ou témoin et re-projeté selon un procédé conforme à l'Etat de la technique.

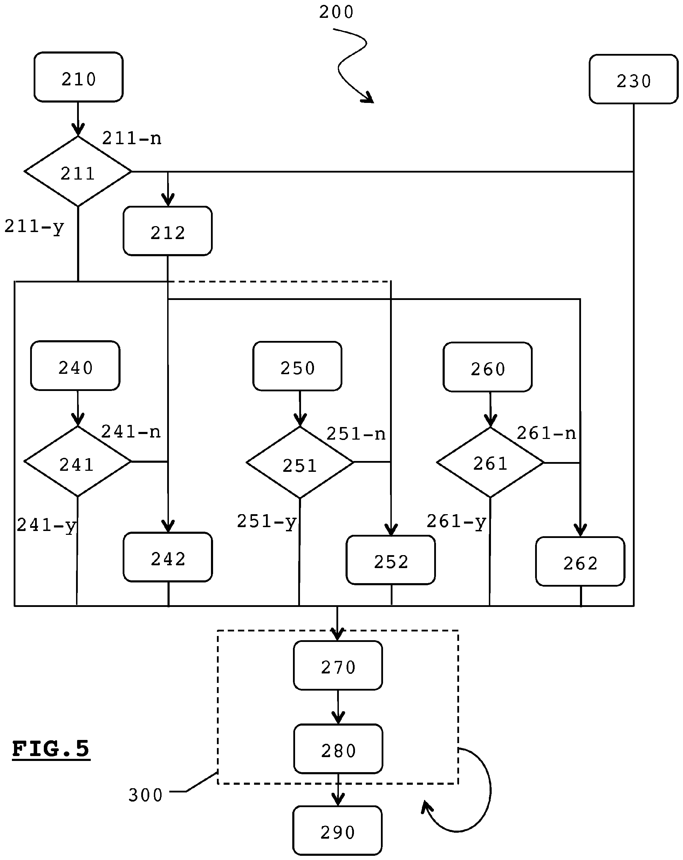

La figure 5 schématise un procédé 200 pour reconstruire un signal physiologique d'un système dynamique artère/tissu/veine d'un organe dans un espace surfacique conforme à l'invention. Comme précisé précédemment, un tel procédé 200 est avantageusement mis en œuvre par des moyens de traitement d'une unité de traitement 4 d'un système d'analyse d'imagerie par Résonance Magnétique ou plus généralement d'un système d'analyse d'imagerie fonctionnelle, tel qu'à titre d'exemples non limitatifs, ceux décrits en liaison avec les figures 1 et 2. Au sens de l'invention et dans tout le document, on entend par « reconstruction d'un signal physiologique » l'élaboration d'un signal physiologique à partir d'une ou plusieurs données expérimentales préalablement acquises par imagerie fonctionnelle. Egalement, au sens de l'invention et dans tout le document, on définit un « espace surfacique » comme un espace décrivant la géométrie du système dynamique artère/tissu/veine d'un organe d'intérêt. Dans le cadre de l'exemple d'application préféré décrit en lien avec le cerveau, un tel espace surfacique, également nommé « surface corticale », consiste en une surface prise dans le ruban cortical, également connu sous la dénomination de « cortex ». Un procédé 200 conforme à l'invention comprend avantageusement une étape 300 pour reconstruire ledit signal physiologique à partir d'une donnée expérimentale d'un volume élémentaire - dit voxel - dudit organe et d'un maillage surfacique décrivant ledit espace surfacique.

Pour rappel, au sens de l'invention et dans tout le document, on entend par « voxel » (contraction anglo-saxonne du terme « *volumetric pixel* »), un volume élémentaire permettant de mesurer la définition d'une image numérique matricielle en trois dimensions. Un tel voxel peut être encore considéré comme un pixel en trois dimensions. Dans tous les cas, un tel voxel peut être considéré comme un parallélépipède rectangle dont la surface fermée est constituée par ses six faces. Egalement, au sens de l'invention et dans tout le document, on entend par « maillage surfacique », toute modélisation géométrique dudit espace surfacique préférentiellement par des éléments proportionnés finis et bien définis. En variante, un tel maillage surfacique peut consister en la modélisation géométrique dudit espace surfacique par des surfaces paramétrés ou des surfaces implicites, comme par exemple des fonctions mathématiques connues sous la dénomination anglo-saxonne « *level-set* ». Ainsi, à titre d'exemple préféré mais non limitatif, on définit un « maillage surfacique » comme un réseau tridimensionnel (3D) composé de nœuds reliés entre eux par des arêtes, c'est-à-dire des segments tridimensionnels délimités par deux nœuds, et formant ainsi un ensemble de faces. Dans le cadre de notre exemple préféré mais non limitatif en lien avec le cerveau, lesdits nœuds peuvent avantageusement consister en des points de l'espace tridimensionnel situés sur, dans ou à proximité du ruban cortical.

Un procédé 200 conforme à l'invention comporte un traitement 300 pour reconstruire un signal physiologique consistant principalement en une étape 270 pour assigner et/ou évaluer une ou plusieurs distributions marginales a posteriori pour ledit signal physiologique que l'on cherche à reconstruire, tel que le signal BOLD en un nœud du maillage. Un tel traitement 300 comporte en outre une étape 280 pour calculer la valeur dudit signal à proprement parler. Pour évaluer une telle distribution marginale a posteriori, il est nécessaire de configurer, manuellement ou automatiquement, l'unité de traitement 4 d'un système d'analyse d'imagerie fonctionnelle, tel que celui décrit précédemment en lien avec les figures 1 et 2. Cette configuration peut être réalisée de préférence par l'unité de traitement 4 elle-même, grâce à ses moyens de traitement, à partir d'un ou plusieurs paramètres de configuration. La configuration peut également se traduire par la constitution d'une bibliothèque d'une ou plusieurs distributions marginales a posteriori, bibliothèque préétablie et mémorisée dans une mémoire de programmes et/ou une mémoire de données, communément dénommées moyens de mémorisation, de ladite unité. L'invention prévoit que ladite bibliothèque peut être enrichie au fur et à mesure de son utilisation, voire délivrée par une unité externe de calcul apte à réaliser ladite configuration à partir du ou des paramètres de configuration et apte à coopérer avec l'unité de traitement pour délivrer ladite bibliothèque.

Un procédé 200 conforme à l'invention peut comporter ainsi des étapes de configuration 240, 250, 260 mises en œuvre préalablement à l'assignation 270, manuellement ou automatiquement, parmi lesquelles sont nécessaires et suffisantes :

- l'assignation 240 de la distribution de probabilité directe des données expérimentales dans ledit espace surfacique sachant les paramètres intervenant dans le problème de reconstruction du signal physiologique du système dynamique artère/tissu/veine pour le voxel considéré ;
- l'assignation 250 d'une distribution de probabilité spatiale a priori dudit signal physiologique par l'introduction d'une information a priori relative à une ou plusieurs caractéristiques des données expérimentales et/ou d'une information a priori relative à une ou plusieurs propriétés du système dynamique artère/tissu/veine ;
- l'assignation 260 d'une distribution de probabilité temporelle a priori dudit signal physiologique par l'introduction d'une information a priori relative à la réponse impulsionnelle dudit système dynamique artère/tissu/veine.

Au sens de l'invention et dans tout le document, le terme de « distribution de probabilité directe » pourra avantageusement être qualifié de « fonction de vraisemblance ».

Par ailleurs, l'invention prévoit qu'un procédé 200 pour reconstruire un signal physiologique conforme à l'invention puisse également comporter une étape de configuration 210 agencée pour permettre l'assignation d'un maillage surfacique décrivant l'espace surfacique de l'organe étudié.

Les étapes de configuration peuvent dépendre du cas d'application considéré. En complément, préalablement aux étapes de configuration 210, 240, 250, 260, un procédé 200 pour reconstruire un signal physiologique conforme à l'invention peut avantageusement et respectivement comprendre des étapes de test 211, 241, 251, 261 pour vérifier la spécification par l'utilisateur de :

- l'assignation 210 d'un maillage surfacique décrivant l'espace surfacique de l'organe étudié ;
- l'assignation 240 de la distribution de probabilité directe des données expérimentales dans ledit espace surfacique sachant les paramètres intervenant dans le problème de reconstruction du signal physiologique du système dynamique artère/tissu/veine pour le voxel considéré ;
- l'assignation 250 d'une distribution de probabilité spatiale a priori dudit signal physiologique par l'introduction d'une information a priori relative à une ou plusieurs caractéristiques des données expérimentales et/ou d'une information a priori relative à une ou plusieurs propriétés du système dynamique artère/tissu/veine ;
- l'assignation 260 d'une distribution de probabilité temporelle a priori dudit signal physiologique par l'introduction d'une information a priori relative à la réponse impulsionnelle dudit système dynamique artère/tissu/veine.

De telles étapes test ou opérations 211, 241, 251, 261 peuvent avantageusement consister à tester la valeur d'un indicateur booléen initialisé ou mis à jour par une ou plusieurs étapes de paramétrage ou de personnalisation, précédemment évoquées, du système d'analyse d'imagerie fonctionnelle dont l'unité de traitement met en œuvre un procédé pour reconstruire un signal physiologique conforme à l'invention ou de toute autre technique mise en œuvre par l'unité de traitement capable de s'assurer d'un tel paramétrage ou d'une telle personnalisation du système d'analyse d'imagerie fonctionnelle.

Si toutes les assignations ont été configurées préalablement (situations symbolisées par les références 211-y, 241-y, 251-y, 261-y en figure 5), l'unité de traitement met en œuvre les étapes subséquentes d'un procédé 200 conforme à l'invention. Autrement, si aucune distribution de probabilité directe des données expérimentales n'a été configurée (situation symbolisée par la référence 241-n en figure 5) au préalable, un procédé 200 conforme à l'invention comporte une étape 242 pour construire ladite distribution de probabilité directe à partir d'informations a priori relatives par exemple, non limitativement, à l'anatomie de l'espace surfacique comprenant la ou les données expérimentales, la nature du signal physiologique à reconstruire, les paramètres d'acquisition de la ou les données expérimentales... etc. Une telle construction peut être réalisée de préférence par l'unité de traitement 4 elle-même, grâce à ses moyens de traitement, à partir d'un ou plusieurs paramètres de construction. La construction peut également se traduire par la constitution d'une bibliothèque d'une ou plusieurs distributions de probabilité directe, bibliothèque préétablie et mémorisée dans une mémoire de programmes de ladite unité, dont l'une desdites distributions de probabilité directe peut être sélectionnée. De manière analogue, si aucune distribution de probabilité spatiale a priori ou temporelle a priori dudit signal physiologique n'a été configurée (situations symbolisées par les références 251-n, 261-n en figure 5) au préalable, un procédé 200 conforme à l'invention comporte respectivement des étapes 252, 262 pour construire une distribution de probabilité spatiale a priori et une distribution de probabilité temporelle a priori. De manière analogue, si aucun maillage surfacique n'a été configuré (situation symbolisée par la référence 211-n en figure 5) au préalable, un procédé 200 conforme à l'invention comporte une étape 212 pour construire un maillage surfacique décrivant ledit espace surfacique à partir d'informations anatomiques relatives à l'organe étudié. En variante ou en complément, si aucune distribution marginale a posteriori pour ledit signal physiologique n'a été configurée ou assignée (situation non représentée sur la figure 5) au préalable, l'invention prévoit qu'un procédé conforme à l'invention puisse comporter une étape pour construire ladite distribution marginale a posteriori.

Les étapes préalablement décrites d'un procédé pour reconstruire un signal physiologique conforme à l'invention ont été décrites en lien avec une approche probabiliste, mais demeurent pertinentes pour la mise en œuvre d'une approche déterministe. Ainsi, l'étape 270 pour évaluer une ou plusieurs distributions marginales a posteriori pour ledit signal physiologique peut consister, selon une approche déterministe, en une étape 270 pour évaluer une ou plusieurs fonctions de coût pour reconstruire ledit signal physiologique. De la même manière, les étapes de configuration 240, 250, 260 mises en œuvre préalablement à l'assignation 270, manuellement ou automatiquement, peuvent consister, selon une approche déterministe, en :

- l'assignation 240 de l'opérateur du modèle direct établissant le lien entre la donnée expérimentale dans le volume élémentaire et ledit signal physiologique dans ledit espace surfacique sachant les paramètres intervenant dans le problème de la reconstruction du signal physiologique du système dynamique artère/tissu/veine pour le voxel considéré ;
- l'assignation 250 d'un opérateur de régularisation spatiale par l'introduction d'une information a priori relative à une caractéristique de la donnée expérimentale et/ou d'une information a priori relative à une ou plusieurs propriétés du système dynamique artère/tissu/veine ;
- l'assignation 260 d'un opérateur de régularisation temporelle par l'introduction d'une information a priori relative à la réponse impulsionnelle dudit système dynamique artère/tissu/veine.

De manière analogue à la distribution de probabilité directe, si aucun opérateur du modèle direct des données expérimentales n'a été configuré (situation symbolisée par la référence 241-n en figure 5) au préalable, un procédé 200 conforme à l'invention comporte une étape 242 pour construire ledit opérateur du modèle direct à partir d'informations a priori relatives par exemple, non limitativement, à l'anatomie de l'espace surfacique comprenant la ou les données expérimentales, la nature du signal physiologique à reconstruire, les paramètres d'acquisition de la ou les données expérimentales... etc. Une telle construction peut être réalisée de préférence par l'unité de traitement 4 elle-même, grâce à ses moyens de traitement, à partir d'un ou plusieurs paramètres de construction. La construction peut également se traduire par la constitution d'une bibliothèque d'un ou plusieurs opérateurs du modèle direct, bibliothèque préétablie et mémorisée dans une mémoire de programmes et/ou une mémoire de données, communément dénommées moyens de mémorisation, de ladite unité, dont l'un desdits opérateurs dudit modèle direct peut être sélectionné. De manière analogue, si aucun opérateur de régularisation spatiale ou temporelle a priori dudit signal physiologique n'a été configuré (situations symbolisées par les références 251-n, 261-n en figure 5) au préalable, un procédé 200 conforme à l'invention comporte respectivement des étapes 252, 262 pour construire un opérateur de régularisation spatiale ou un opérateur de régularisation temporelle.

Des premier et deuxième exemples d'implémentation d'un tel procédé 200 pour reconstruire un signal physiologique, respectivement suivant des approches déterministe et probabiliste, seront avantageusement mais non limitativement décrits dans la suite du document, en lien avec la figure 5 permettant d'illustrer un procédé 200 pour un signal physiologique conforme à l'invention. A ce titre, une donnée expérimentale, sous la forme d'un volume fonctionnel V est acquise à chaque instant $t$.

Selon un premier exemple d'implémentation suivant une approche déterministe, un modèle direct expérimental a été choisi et défini d'une part pour traduire le comportement physiologique du signal BOLD, en exprimant notamment la propagation d'une activité neuronale d'une colonne corticale à ses colonnes voisines de sorte que : à chaque instant $t$, un signal physiologique cortical $A$ en un nœud $n$ d'un maillage surfacique influe sur ses nœuds voisins $m$ dudit maillage surfacique selon un poids $\omega_{geodesique}(n,m)$ inversement proportionnel à la distance géodésique, c'est-à-dire le long de la surface, les séparant. Par ailleurs, ledit modèle direct expérimental a été choisi et défini d'autre part pour modéliser les phénomènes physiques intervenant au cours de l'acquisition d'une ou plusieurs données expérimentales par un système d'imagerie par Résonance Magnétique, en décrivant notamment l'effet de volume partiel, de sorte que : à chaque instant $t$, un signal physiologique cortical $A$ en un nœud $n$ d'un maillage surfacique influe sur les voxels $v$ environnant dudit nœud $n$, c'est-à-dire normalement à la surface, selon un poids $\omega_{normal}(v,n)$ maximal dans la matière grise et inversement proportionnel à la distance entre lesdits voxels et la matière grise dès lors que lesdits voxels sont positionnés dans la matière blanche et le liquide céphalo-rachidien. Aussi, un modèle direct, sous forme d'un modèle de pondération normal et géodésique, peut s'écrire sous la forme du système d'équations suivant :

$$\begin{cases} V(v,t) = \sum_{n=1}^{N_n} \omega_{normal}(v,n)A(n,t) \\ A(n,t) = \sum_{m=1}^{N_n} \omega_{geodesique}(n,m)A(m,t) \end{cases} \Rightarrow V(v,t) = \sum_{m=1}^{N_n} M(v,m)A(m,t)$$

Ainsi, un procédé conforme à l'invention comporte une étape de configuration 240 pour assigner un opérateur du modèle direct $M$ sous la forme d'une matrice de taille $N_v \times N_n$, où $N_v$ est le nombre de voxels $v$ contenus dans une donnée expérimentale $V$, également connue sous le terme de volume fonctionnel, et $N_n$ le nombre de nœuds du maillage surfacique, établissant le lien entre la donnée expérimentale $V$ dans le volume élémentaire $v$ et ledit signal physiologique *A* dans ledit espace surfacique, sachant les poids $\omega_{geodesique}(n,m)$ et $\omega_{normal}(v,n)$.

Par ailleurs, un procédé 200 conforme à l'invention comporte une étape de configuration 250 pour assigner un opérateur de régularisation spatiale $E_{spatial}(A)$ du signal physiologique à reconstruire par l'introduction d'une information a priori relative à une caractéristique de la donnée expérimentale et/ou d'une information a priori relative à une propriété du système dynamique artère/tissu/veine, tel que :

$$E_{spatial}(A) = \lambda_D Tr((DA)^t (DA))$$

où $\lambda_D$ est un coefficient de régularisation spatiale,
*A* est la matrice du signal physiologique à reconstruire, et *D* est la matrice de régularisation spatiale s'écrivant sous la forme :

$$D_{i,j} = \frac{\delta_{j,c_{i_1}} - \delta_{j,c_{i_2}}}{d_g(n_{c_{i_1}}, n_{c_{i_2}}) D_{norm}(n_{c_{i_1}}, n_{c_{i_2}})} \quad avec \quad \left| \begin{array}{l} \delta_{i,j} = \begin{cases} 1, \ si \ i = j \\ 0, \ dans \ le \ cas \ contraire \end{cases} \\ D_{norm}(n_{c_{i_1}}, n_{c_{i_2}}) = \sqrt{\dfrac{\#v(n_{c_{i_1}})\#v(n_{c_{i_2}})}{\#v(n_{c_{i_1}}) + \#v(n_{c_{i_2}})}} \end{array} \right.$$

où $\delta_{i,j}$ est le symbole de Kronecker, $d_g(n_{c_{i1}}, n_{c_{i2}})$ est la distance géodésique entre deux nœuds aux extrémités d'une arête du maillage surfacique, $D_{norm}(n_{c_{i1}}, n_{c_{i2}})$ est un terme de normalisation avec $n_{c_{i1}}$ et $n_{c_{i2}}$ correspondant aux deux nœuds aux extrémités d'une arête $c_i$ du maillage surfacique et $\#v(n_{c_{i1}})$ et $\#v(n_{c_{i2}})$ correspondant respectivement au nombre de voisins directs des nœuds $n_{ci1}$ et $n_{ci2}$.

En outre, un procédé 200 conforme à l'invention comporte une étape de configuration 260 pour assigner un opérateur de régularisation temporelle $E_{temporel}(A)$ par l'introduction d'une information a priori relative à la réponse impulsionnelle dudit système dynamique artère/tissu/veine, tel que :

$$E_{temporel}(A) = \lambda_T Tr(AT^t TA^t)$$

où $\lambda_T$ est un coefficient de régularisation temporelle,
*A* est la matrice du signal physiologique à reconstruire, et *T* est la matrice de régularisation temporelle s'écrivant sous la forme :

$$T_{ordre2} = \frac{1}{\Delta_T^2} \begin{pmatrix} 1 & -2 & 1 & 0 & \cdots & 0 \\ 0 & 1 & -2 & 1 & \ddots & \vdots \\ \vdots & \ddots & \ddots & \ddots & \ddots & 0 \\ 0 & \cdots & 0 & 1 & -2 & 1 \end{pmatrix}$$

où $\Delta_T$ est un intervalle de temps entre deux acquisitions de deux volumes fonctionnels.

De plus, un procédé 200 conforme à l'invention comporte une étape de configuration 270 pour assigner une fonction de coût $E(V,A)$ pour ledit signal physiologique A en un nœud dudit maillage, tel que :

$$E(V,A) = \frac{Tr((V - MA)^t R^{-1}(V - MA))}{2} + \lambda_D Tr((DA)^t (DA)) + \lambda_T Tr(AT^t TA^t)$$

où $E_{attache}(V,A) = \dfrac{Tr((V - MA)^t R^{-1}(V - MA))}{2}$ est le terme d'attache aux données mesurant l'écart entre le modèle direct appliqué au signal physiologique *A* et les données expérimentales *V* en considérant l'hypothèse d'un bruit gaussien, avec *M* l'opérateur du modèle direct et $R^{-1}$ la matrice de covariance du bruit ;

$E_{spatial}(A)=\lambda_D Tr((DA)^t(DA))$ est l'opérateur de régularisation spatiale préalablement configuré, $E_{temporel}(A)=\lambda_T Tr(AT^tTA^t)$ est l'opérateur de régularisation temporelle préalablement configuré.

Enfin, à des fins d'optimisation, un procédé 200 conforme à l'invention comporte une étape 280 pour évaluer ladite fonction de coût $E(V,A)$ pour ledit signal physiologique $A$ en un nœud dudit maillage. Une telle étape 280 pour évaluer ladite fonction de coût $E(V,A)$ consiste à minimiser une telle fonction de coût $E(V,A)$ selon le signal physiologique $A$, en minimisant, voire annulant, le gradient de l'énergie totale, consistant à résoudre l'équation suivante :

$$\nabla E(V,A) = (M^t R^{-1} M + 2\lambda_D D^t D)A + 2\lambda_T A T^t T - M^t R^{-1} V = 0$$

Finalement, l'étape 280 pour évaluer ladite fonction de coût $E(V,A)$ consiste à résoudre le système suivant, par la mise en œuvre par des moyens de traitement d'une unité de traitement 4 d'un système d'analyse d'imagerie fonctionnelle, tel que celui décrit en lien avec les figures 1 et/ou 2, de l'algorithme des gradients conjugués linéaires :

$$HA + AG = K \; avec \begin{cases} H = M^t R^{-1} M + 2\lambda_D D^t D \\ G = 2\lambda_T T^t T \\ K = M^t R^{-1} V \end{cases}$$

Selon un deuxième exemple d'implémentation suivant une approche probabiliste, un modèle direct expérimental a été choisi et défini d'une part pour traduire le comportement physiologique du signal BOLD, en exprimant notamment la propagation d'une activité neuronale d'une colonne corticale à ses colonnes voisines de sorte que : à chaque instant $t$, un signal physiologique cortical $A$ en un nœud $n$ d'un maillage surfacique influe sur ses nœuds voisins $m$ dudit maillage surfacique selon un poids $\omega_{geodesique}(n,m)$ inversement proportionnel à la distance géodésique, c'est-à-dire le long de la surface, les séparant. Par ailleurs, ledit modèle direct expérimental a été choisi et défini d'autre part pour modéliser les phénomènes physiques intervenant au cours de l'acquisition d'une ou plusieurs données expérimentales par un système d'imagerie par Résonance Magnétique, en décrivant notamment l'effet de volume partiel, de sorte que : à chaque instant $t$, un signal physiologique cortical $A$ en un nœud $n$ d'un maillage surfacique influe sur les voxels $v$ environnant dudit nœud $n$, c'est-à-dire normalement à la surface, selon un poids $\omega_{normal}(v,n)$ maximal dans la matière grise et inversement proportionnel à la distance entre lesdits voxels et la matière grise dès lors que lesdits voxels sont positionnés dans la matière blanche et le liquide céphalo-rachidien. Aussi, un modèle direct, sous forme d'un modèle de pondération normal et géodésique, peut s'écrire sous la forme du système d'équations suivant :

$$\begin{cases} V(v,t) = \sum_{n=1}^{N_n} \omega_{normal}(v,n)A(n,t) \\ A(n,t) = \sum_{m=1}^{N_n} \omega_{geodesique}(n,m)A(m,t) \end{cases} \Rightarrow V(v,t) = \sum_{m=1}^{N_n} M(v,m)A(m,t)$$

Ainsi, un procédé conforme à l'invention comporte une étape de configuration 240 pour assigner une distribution de probabilité $M$, sous la forme d'une matrice de taille $N_v \times N_n$, où $N_v$ est le nombre de voxels $v$ contenus dans une donnée expérimentale $V$, également connue sous le terme de volume fonctionnel, et $N_n$ le nombre de nœuds du maillage surfacique, de la donnée expérimentale $V$ dans ledit espace surfacique, sachant les poids $\omega_{geodesique}(n,m)$ et $\omega_{normal}(v,n)$ pour le voxel considéré $v$.

Par ailleurs, un procédé 200 conforme à l'invention comporte une étape de configuration 250 pour assigner une distribution de probabilité spatiale a priori dudit signal physiologique $p_{spatial}(A)$ par l'introduction d'une information a priori relative à une caractéristique de la donnée expérimentale et/ou d'une information a priori relative à une propriété du système dynamique artère/tissu/veine, tel que :

$$p_{spatial}(A) = \frac{1}{Z_{spatial}} e^{-\lambda_D Tr((DA)^t(DA))}$$

où $\lambda_D$ est un coefficient de régularisation spatiale, $\dfrac{1}{Z_{spatial}}$ est un terme de normalisation, $A$ est la matrice du signal physiologique à reconstruire, et $D$ est la matrice de régularisation spatiale s'écrivant sous la forme :

$$D_{i,j} = \frac{\delta_{j,c_{i1}} - \delta_{j,c_{i2}}}{d_g(n_{c_{i1}}, n_{c_{i2}}) D_{norm}(n_{c_{i1}}, n_{c_{i2}})} \quad avec \left| \begin{array}{l} \delta_{i,j} = \begin{cases} 1, \; si \; i = j \\ 0, \; dans \; le \; cas \; contraire \end{cases} \\[2ex] D_{norm}(n_{c_{i1}}, n_{c_{i2}}) = \sqrt{\dfrac{\# v(n_{c_{i1}}) \# v(n_{c_{i2}})}{\# v(n_{c_{i1}}) + \# v(n_{c_{i2}})}} \end{array} \right.$$

où $\delta_{i,j}$ est le symbole de Kronecker, $d_g(n_{c_{i1}}, n_{c_{i2}})$ est la distance géodésique entre deux nœuds aux extrémités d'une arête du maillage surfacique, $D_{norm}(n_{c_{i1}}, n_{c_{i2}})$ est un terme de régularisation spatiale A avec $n_{c_{i1}}$ et $n_{c_{i2}}$ correspondant aux deux nœuds aux extrémités d'une arête $c_i$ du maillage surfacique et **$\# v(n_{c_{i1}})$** et $\# v(n_{c_{i2}})$ correspondant respectivement au nombre de voisins directs des nœuds $n_{c_{i1}}$ et $n_{c_{i2}}$.

En outre, un procédé 200 conforme à l'invention comporte une étape de configuration 260 pour assigner une distribution de probabilité temporelle a priori dudit signal physiologique $p_{temporel}(A)$ par l'introduction d'une information a priori relative à la réponse impulsionnelle dudit système dynamique artère/tissu/veine, tel que :

$$p_{temporel}(A) = \frac{1}{Z_{temporel}} e^{-\lambda_T Tr(AT^t TA^t)}$$

où $\lambda_T$ est un coefficient de régularisation temporelle, $\dfrac{1}{Z_{temporel}}$ est un terme de normalisation, $A$ est la matrice du signal physiologique à reconstruire, et $T$ est la matrice de régularisation temporelle s'écrivant sous la forme :

$$T_{ordre2} = \frac{1}{\Delta_T^2} \begin{pmatrix} 1 & -2 & 1 & 0 & \cdots & 0 \\ 0 & 1 & -2 & 1 & \ddots & \vdots \\ \vdots & \ddots & \ddots & \ddots & \ddots & 0 \\ 0 & \cdots & 0 & 1 & -2 & 1 \end{pmatrix}$$

où $\Delta_T$ est un intervalle de temps entre deux acquisitions de deux volumes fonctionnels.

De plus, un procédé 200 conforme à l'invention comporte une étape de configuration 270 pour assigner une distribution marginale a posteriori $p(A|V)$ pour ledit signal physiologique $A$ en un nœud dudit maillage, tel que :

$$p(A|V) \propto e^{-\left( \frac{Tr((V-MA)^t R^{-1}(V-MA))}{2} + \lambda_D Tr((DA)^t(DA)) + \lambda_T Tr(AT^t TA^t) \right)}$$

où $e^{-\left( \frac{Tr((V-MA)^t R^{-1}(V-MA))}{2} \right)}$ correspond à la fonction de vraisemblance, correspond à la distribution de probabilité spatiale a priori, et $e^{-(\lambda_T Tr(AT^t TA^t))}$ correspond à la distribution de probabilité temporelle a priori, lesdites fonction de vraisemblance, distribution de probabilité spatiale a priori et distribution de probabilité temporelle a priori préalablement assignées à une constante multiplicative près.

Enfin, à des fins d'optimisation, un procédé 200 conforme à l'invention comporte une étape 280 pour évaluer la distribution marginale a posteriori $p(A|V)$ pour ledit signal physiologique $A$ en un nœud dudit maillage. Une telle étape 280 pour évaluer ladite distribution marginale a posteriori $p(A|V)$ consiste à maximiser la distribution marginale a posteriori $p(A|V)$ selon le signal physiologique $A$, en appliquant l'Estimateur du Maximum A Posteriori, tel que :

$$\arg\max_A(p(A|V)) = \arg\max_A(e^{-\left( \frac{Tr((V-MA)^t R^{-1}(V-MA))}{2} + \lambda_D Tr((DA)^t(DA)) + \lambda_T Tr(AT^t TA^t) \right)})$$

soit :

$$\arg\max_A(p(A|V)) = \arg\min_A\left(\frac{Tr((V-MA)^t R^{-1}(V-MA))}{2} + \lambda_D Tr((DA)^t(DA)) + \lambda_T Tr(AT^tTA^t)\right)$$

Finalement, l'étape 280 pour évaluer la distribution marginale a posteriori *p(A|V)* consiste à résoudre le système suivant, par la mise en œuvre par des moyens de traitement d'une unité de traitement 4 d'un système d'analyse d'imagerie fonctionnelle, tel que celui décrit en lien avec les figures 1 et/ou 2, de l'algorithme des gradients conjugués linéaires :

$$HA + AG = K \ avec \begin{cases} H = M^t R^{-1} M + 2\lambda_D D^t D \\ G = 2\lambda_T T^t T \\ K = M^t R^{-1} V \end{cases}$$

En outre, un procédé 200 pour reconstruire un signal physiologique peut comporter une étape 230 pour produire ladite donnée expérimentale à partir d'une acquisition d'un signal par imagerie fonctionnelle. L'acquisition d'une ou plusieurs données expérimentales, avantageusement des signaux, par imagerie fonctionnelle, plus particulièrement Imagerie de Résonance Magnétique, peut être effectuée en échantillonnant régulièrement un volume parallélépipédique selon un plan de coupe donné. Les données expérimentales, également connues sous la dénomination d'« images », en deux dimensions obtenues sont constituées de pixels dotés d'une épaisseur, correspondant à l'épaisseur de la coupe et appelés voxels. En IRM, plus particulièrement en IRMf, une telle acquisition peut s'effectuer au moyen d'une ou plusieurs séquences définies par des paramètres d'acquisition tels que, par exemple, le temps d'écho *TE,* le temps de répétition *TR,* l'angle de bascule $\alpha$ ou encore le temps d'inversion *TI*. A titre d'exemple préféré mais non limitatif, une séquence d'acquisition écho planaire (également connue sous la terminologie anglo-saxonne *« echo planar imaging »* ou l'abréviation *« EPI »*) pourra être employée. En variante, une séquence d'écho de gradient, généralement prévue dans tous les systèmes d'imagerie, pourra également être employée.

Par ailleurs, comme précisé précédemment, notamment dans l'exemple décrit en lien avec les figures 1 et 2, un système d'analyse d'imagerie fonctionnelle conforme à l'invention peut comporter des moyens de restitution 5 du signal physiologique reconstruit à un utilisateur 6 dudit système, lesdits moyens de restitution 5 coopérant avec l'unité de traitement 4. De tels moyens de restitution 5 permettent de disposer d'un rendu avantageusement graphique, sonore ou autre et peuvent comporter par exemple un écran ou des haut-parleurs. Ainsi, en variante ou en complément, un procédé 200 conforme à l'invention peut avantageusement comporter une étape subséquente 290 pour déclencher une restitution du signal physiologique reconstruit selon un format approprié. Selon l'exemple d'application préférée mais non limitative en lien avec le cerveau, le signal physiologique reconstruit est avantageusement le signal BOLD. Par exemple, le ou les signaux physiologiques reconstruits peuvent avantageusement prendre la forme d'une ou plusieurs données surfaciques dynamiques. En effet, pour chaque nœud du maillage surfacique en lequel un signal physiologique est reconstruit, en chaque instant, c'est-à-dire pour chacun des instants correspondant à une donnée expérimentale, les moyens de restitution d'un système d'analyse par imagerie fonctionnelle, tel que, par exemple, celui décrit précédemment en lien avec les figures 1 et 2, peuvent restituer une valeur correspondant à la valeur dudit signal physiologique en ce nœud et en cet instant. Ainsi, la ou lesdites valeurs dudit signal physiologique peuvent être restituées par les moyens de restitution dudit système d'analyse d'imagerie fonctionnelle sous différentes formes, notamment une ou plusieurs représentations graphiques de différentes natures, telles qu'à titre d'exemples non limitatifs, un ou plusieurs décours temporels, une ou plusieurs textures dynamiques ou statiques.

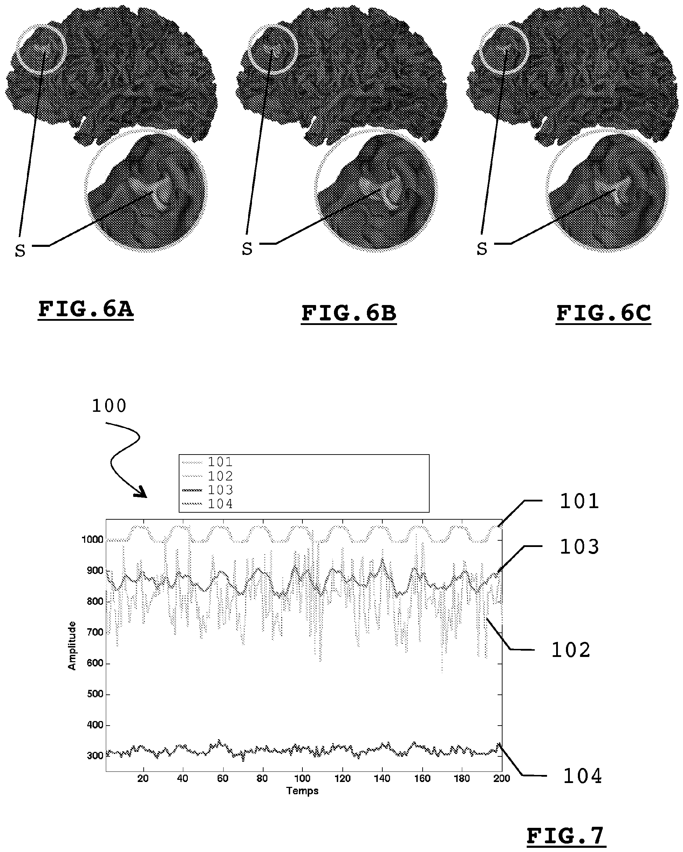

Au sens de l'invention et dans tout le document, on entend par « décours temporel » l'évolution d'un signal physiologique dans le temps en un point prédéterminé, tel qu'un nœud ou un voxel d'intérêt, représentée par une courbe d'amplitude en fonction du temps. La figure 7 présente notamment un ensemble 100 de plusieurs exemples de tels décours temporels 101, 102, 103, 104 en un même nœud. La courbe 101 illustre notamment le décours temporel d'un signal physiologique original ou témoin en un nœud. La courbe 102 quant à elle montre une représentation graphique du décours temporel du même signal physiologique bruité d'un voxel contenant ledit nœud. A son tour, la courbe 103 illustre notamment le décours temporel du signal physiologique reconstruit par un procédé conforme à l'invention en le même nœud. Enfin, la courbe 104 montre une représentation graphique du décours temporel du signal physiologique reconstruit par un procédé selon l'Etat de la Technique en le même nœud. Comme le montre l'exemple décrit en lien avec la courbe 102 de la figure 7, à partir des données expérimentales acquises en IRMf, l'utilisateur ne peut extraire aucune information pertinente relative au signal physiologique « à l'œil nu », et ce principalement du fait de la présence d'un bruit important. Les variations d'intérêt du signal étant très faibles, un niveau de bruit, quand bien même faible, suffit à interférer avec le signal et empêche d'obtenir une information pertinente relative au signal physiologique. Comme le montre l'exemple décrit en lien avec la courbe 103 de la figure 7, un procédé conforme à l'invention permet non seulement d'améliorer la qualité d'un signal physiologique reconstruit en comparaison avec la restitution du même signal physiologique reconstruit par un procédé selon l'Etat de la Technique, tel que l'atteste la courbe 104 de la figure 7, et offre également à l'utilisateur la possibilité de relever, sans traitement supplémentaire, des informations pertinentes contenues au sein dudit signal physiologique reconstruit. Par exemple, en réduisant le bruit contenu dans les données expérimentales, un tel procédé pourrait permettre à l'utilisateur, en affichant d'une part le paradigme expérimental convolué à la fonction de réponse hémodynamique 101, et d'autre part le décours temporel du signal physiologique reconstruit par ledit procédé en différents nœuds d'intérêt du maillage surfacique, de déceler des relations de corrélation entre ledit signal physiologique reconstruit et le paradigme expérimental convolué à la fonction de réponse hémodynamique. Ainsi que l'atteste la figure 7, le décours temporel du signal physiologique reconstruit par un procédé conforme à l'invention, représenté par la courbe 103 de ladite figure 7, est bien plus « propre » que le décours temporel du signal physiologique expérimental, représenté par la courbe 102 de ladite figure 7, puisqu'un procédé conforme à l'invention permet d'assurer une corrélation spatiale entre les signaux physiologiques, tandis qu'il permet de diminuer l'impact du bruit et d'améliorer la visibilité des variations d'intérêt dudit signal physiologique reconstruit.

Egalement, au sens de l'invention et dans tout le document, on définit une « texture statique » comme l'ensemble des valeurs prises par un signal physiologique en chacun des nœuds du maillage surfacique à un instant t. De manière analogue, on définit une « texture dynamique » comme une série temporelle de textures statiques pour une pluralité d'instants t. Les figures 6A à 6C présentent notamment un ensemble 100 de plusieurs exemples de telles textures statiques. Tout d'abord, la figure 6A illustre notamment une texture statique d'un signal physiologique reconstruit S par un procédé conforme à l'invention, en lien avec le cerveau et le signal BOLD. De la même manière, la figure 6B montre une représentation graphique d'une texture statique du même signal physiologique original ou témoin S, en lien également avec le cerveau et le signal BOLD. Enfin, la figure 6C illustre notamment une texture statique d'un signal physiologique reconstruit S par un procédé selon l'Etat de la Technique, en lien avec le cerveau et le signal BOLD. Selon la figure 6A, en comparaison avec le signal BOLD expérimental décrit en lien avec la figure 6B et le signal BOLD reconstruit par un procédé selon l'Etat de la Technique, le signal BOLD reconstruit et restitué par un procédé conforme à l'invention apparaît bien mieux localisé spatialement et l'amplitude dudit signal BOLD est clairement bien mieux restaurée qu'au regard de l'Etat de la Technique.

En variante ou en complément, pour améliorer la qualité d'une ou plusieurs données ou signaux expérimentaux obtenus et acquis par imagerie fonctionnelle, plus précisément par Résonance Magnétique Fonctionnelle, mais également la qualité et la robustesse du signal physiologique reconstruit, un procédé 200 conforme à l'invention peut également comporter une ou plusieurs étapes préalables (non représentées sur les figures) de prétraitement de la donnée expérimentale, ladite étape étant agencée pour corriger ladite donnée expérimentale.

En effet, l'imagerie par Résonance Magnétique, comme toutes les autres techniques d'imagerie médicale, n'échappe pas à la constitution d'artéfacts. Les artéfacts sont des images observables ne représentant aucune réalité anatomique ou physique. Bien souvent, on cherche à les éviter ou à les minimiser en modifiant certains paramètres d'acquisition ou de reconstruction. De tels artefacts peuvent être de différentes natures. En outre, le système d'acquisition d'imagerie par Résonance Magnétique Fonctionnelle, plus généralement d'imagerie fonctionnelle, peut également influencer les données expérimentales obtenues. En effet, les données expérimentales IRMf, généralement sous la forme d'images, résultent de compromis entre de nombreux critères interdépendants, tels que, non limitativement, la durée de l'acquisition, le rapport signal sur bruit, la taille du volume acquis, la résolution spatiale ou encore la résolution temporelle.

A titre d'exemples non limitatifs, de telles étapes pour corriger une ou plusieurs données expérimentales peuvent consister en :

- une étape de correction de mouvements (réalignement), notamment de la tête, si le patient ne demeure pas suffisamment immobile au cours de l'acquisition d'une séquence, au moyen de deux étapes successives comprenant une étape d'estimation de six paramètres correspondant aux transformations rigides (trois translations et trois rotations selon les trois axes de l'espace euclidien) et une étape de transformation des données estimées au moyen de méthodes d'interpolation trilinéaire, sinusoïdale ou B-spline ;
- une étape de correction du décalage temporel ou de recalage temporel intra-coupe (également connue sous la dénomination anglo-saxonne de « slice-timing »), au moyen d'une étape d'interpolation temporelle, permettant de considérer toutes les coupes d'une même donnée expérimentale comme acquises au même instant. En effet, l'acquisition des coupes d'une donnée expérimentale n'étant pas faite au même instant et la durée de ladite acquisition étant fonction du temps de répétition TR, les signaux compris au sein d'une même coupe peuvent démontrer un décalage dans le temps ;

- une étape de recalage anatomo-fonctionnel, permettant la mise en correspondance des données expérimentales (également connues sous la terminologie de « données fonctionnelles ») et des données anatomiques d'un sujet ;
- une étape de correction de biais et de distortions géométriques dus aux inhomogénéités de champs magnétiques B1 appliqués au sein de l'appareil d'imagerie par Résonance Magnétique qui affectent couramment les signaux expérimentaux par Résonance Magnétique.

Par ailleurs, comme précisé précédemment, l'invention prévoit qu'un procédé conforme à l'invention puisse comporter une étape préalable (non représentée sur les figures) de prétraitement du maillage surfacique, ladite étape étant agencée pour recaler ledit maillage surfacique. En effet, il peut être nécessaire que la ou les données expérimentales et le maillage surfacique soient mis en correspondance, afin de reconstruire finalement le signal physiologique. Aussi, le maillage surfacique peut être avantageusement replacé dans le référentiel de la ou des données expérimentales. A titre d'exemple non limitatif, l'étape pour recaler ledit maillage surfacique peut comporter une ou plusieurs étapes de recalage similaires à celles décrites précédemment, telles que, par exemple, une étape de recalage rigide.

L'invention concerne en outre un procédé 200 pour produire une reconstruction d'un signal physiologique d'une région d'intérêt. On entend par « région d'intérêt » toute région s'étendant sur au moins un voxel d'intérêt. Néanmoins, une région d'intérêt ne saurait être limitée à un seul voxel, mais peut comprendre une pluralité de voxels, avantageusement sélectionnés manuellement ou automatiquement. Selon l'invention, ledit signal physiologique peut être reconstruit en au moins deux nœuds concernés par ladite région d'intérêt pour chacun desdits nœuds à partir d'une ou plusieurs données expérimentales au moyen d'un tel procédé 200 conforme à l'invention, tel que celui décrit précédemment, notamment en lien avec la figure 5, ledit procédé 200 étant mis en œuvre par les moyens de traitement de l'unité de traitement 4 d'un système d'analyse d'imagerie fonctionnelle, plus particulièrement d'imagerie par Résonance Magnétique, selon les figures 1 et/ou 2.

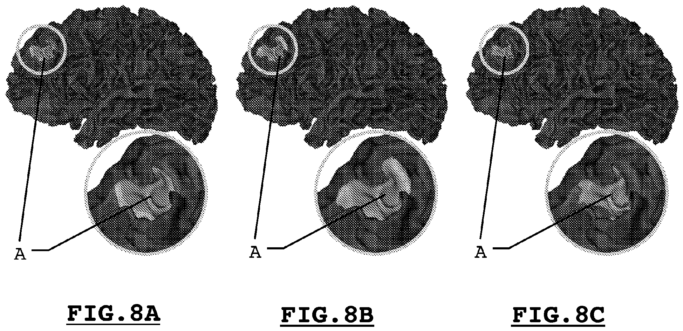

En outre, comme précisé précédemment, notamment dans ledit exemple décrit en lien avec les figures 1 et 2, un système d'analyse d'imagerie fonctionnelle conforme à l'invention peut comporter des moyens de restitution 5 du signal physiologique reconstruit à un utilisateur 6 dudit système, lesdits moyens de restitution 5 coopérant avec l'unité de traitement 4. De tels moyens de restitution 5 permettent de disposer d'un rendu avantageusement graphique, sonore ou autre et peuvent comporter, par exemple, un écran ou des haut-parleurs. Ainsi, en variante ou en complément, un procédé 200 conforme à l'invention peut avantageusement comprendre une étape subséquente pour déclencher la restitution du signal physiologique reconstruit en un ou plusieurs nœuds du maillage pour chaque voxel de la région d'intérêt et générer une image à partir de la reconstruction dudit signal physiologique sous la forme d'une carte d'activité fonctionnelle selon un format approprié. Une telle génération de carte d'activité fonctionnelle est mise en œuvre grâce à un deuxième procédé consécutif à un procédé pour construire un signal physiologique conforme au premier objet de l'invention et s'appuie sur des méthodes utilisant, par exemple, un modèle linéaire général (également connu sous la terminologie et l'abréviation anglo-saxonnes « General Linear Model - GLM »). Dans le cadre de l'exemple d'application préféré en lien avec le cerveau, une telle carte d'activité fonctionnelle permet la détection d'activations neuronales à partir de la reconstruction du signal BOLD. Les figures 8A, 8B et 8C présentent trois exemples de cartes d'activité fonctionnelle générées dans le cadre de l'exemple d'application préférée mais non limitative, en l'espèce le cerveau. La figure 8A illustre une carte d'activité neuronale générée et restituée à partir d'un signal BOLD reconstruit selon un procédé conforme à l'invention. La figure 8B, quant à elle, illustre une carte d'activité neuronale à partir d'un signal BOLD original ou témoin. Enfin, la figure 8C illustre une carte d'activité neuronale générée et restituée à partir d'un signal BOLD reconstruit selon un procédé conforme à l'État de la technique. Selon la figure 8A, la zone active A de la carte d'activité neuronale apparaît manifestement bien plus nette et mieux obtenue que celle obtenue par un procédé selon l'Etat de la Technique, comme le montre la comparaison des figures 8A et 8C au regard du référentiel témoin décrit par la figure 8B.

Grâce aux nouvelles reconstructions d'un signal physiologique et/ou aux restitutions dudit signal physiologique reconstruit présentées précédemment, l'invention permet de mettre à la disposition d'un utilisateur, éventuellement praticien, tout un ensemble d'informations pertinentes et cohérentes, informations disponibles grâce à l'utilisation d'un procédé conforme à l'invention. Cette mise à disposition est rendue possible par une adaptation de l'unité de traitement 4 selon les figures 1 ou 2, en ce que les moyens de traitement d'une telle unité de traitement 4 mettent en œuvre un procédé pour reconstruire un signal physiologique d'un voxel ou d'une région d'intérêt comportant notamment la reconstruction dudit signal physiologique à partir d'une ou plusieurs données expérimentales d'un voxel dudit organe et d'un maillage surfacique décrivant ledit espace surfacique. Une telle mise en œuvre est avantageusement rendue possible par le chargement ou l'enregistrement, au sein de moyens de mémorisation, éventuellement comportés au sein de l'unité de traitement 4, coopérant avec lesdits moyens de traitement, d'un produit programme d'ordinateur. Ce dernier comporte en effet des instructions interprétables et/ou exécutables par lesdits moyens de traitement. L'interprétation ou l'exécution desdites instructions provoque ou déclenche automatiquement la mise en œuvre d'un procédé 200 conforme à l'invention. Les moyens pour communiquer avec le monde extérieur de ladite unité de traitement peuvent délivrer un signal physiologique, tel qu'à titre d'exemple préféré mais non limitatif le signal BOLD, selon un format approprié à des moyens de restitution aptes à la restituer à un utilisateur 6, ledit signal physiologique reconstruit pouvant être avantageusement restitué sous la forme, par exemple, de décours temporels, de textures statiques ou dynamiques, ou encore de cartes d'activité fonctionnelle, tels que les exemples précédemment décrits et illustrés par les figures 6A, 7 et 8A. Grâce à l'invention, les informations délivrées sont plus nombreuses, cohérentes, reproductibles et justes.

## Revendications

1. Procédé (200) pour reconstruire un signal physiologique d'un système dynamique artère/tissu/veine d'un organe dans un espace surfacique, ledit procédé (200) étant mis en œuvre par des moyens de traitement d'une unité de traitement (4) d'un système d'analyse d'imagerie fonctionnelle, et comportant une étape (270) pour reconstruire ledit signal physiologique à partir d'une donnée expérimentale d'une région d'intérêt comportant un volume élémentaire - dit voxel - dudit organe et d'un maillage surfacique décrivant ledit espace surfacique, ledit procédé étant **caractérisé en ce que** ladite étape consiste à évaluer (280) suivant une méthode de résolution d'un problème inverse une distribution marginale a posteriori pour ledit signal physiologique en un nœud dudit maillage par :

   - l'assignation (240) d'une distribution de probabilité directe de la donnée expérimentale dans ledit espace surfacique sachant les paramètres intervenant dans le problème de reconstruction du signal physiologique du système dynamique artère/tissu/veine pour le voxel considéré ;
   - l'assignation conjointe (250) d'une distribution de probabilité spatiale a priori dudit signal physiologique par l'introduction d'une information a priori relative à une caractéristique de la donnée expérimentale et/ou d'une information a priori relative à une propriété du système dynamique artère/tissu/veine ;
   - l'assignation conjointe (260) d'une distribution de probabilité temporelle a priori dudit signal physiologique par l'introduction d'une information a priori relative à la réponse impulsionnelle dudit système dynamique artère/tissu/veine.

2. Procédé (200) pour reconstruire un signal physiologique d'un système dynamique artère/tissu/veine d'un organe dans un espace surfacique, ledit procédé (200) étant mis en œuvre par des moyens de traitement d'une unité de traitement (4) d'un système d'analyse d'imagerie fonctionnelle, et comportant une étape pour reconstruire le signal physiologique à partir d'une donnée expérimentale d'une région d'intérêt comportant un volume élémentaire - dit voxel - et d'un maillage surfacique décrivant ledit espace surfacique, ledit procédé étant **caractérisé en ce que** ladite étape consiste à évaluer (270) suivant une méthode de résolution d'un problème inverse une fonction de coût pour ledit signal physiologique en un nœud dudit maillage par :

   - l'assignation (240) d'un opérateur du modèle direct établissant le lien entre la donnée expérimentale dans le volume élémentaire et ledit signal physiologique dans ledit espace surfacique sachant les paramètres intervenant dans le problème de la reconstruction du signal physiologique du système dynamique artère/tissu/veine pour le voxel considéré ;
   - l'assignation conjointe (250) d'un opérateur de régularisation spatiale par l'introduction d'une information a priori relative à une caractéristique de la donnée expérimentale et/ou d'une information a priori relative à une propriété du système dynamique artère/tissu/veine ;
   - l'assignation conjointe (260) d'un opérateur de régularisation temporelle par l'introduction d'une information a priori relative à la réponse impulsionnelle dudit système dynamique artère/tissu/veine.

3. Procédé (200) selon l'une quelconque des revendications précédentes, comportant en outre une étape (230) pour produire ladite donnée expérimentale à partir d'une acquisition d'un signal par imagerie fonctionnelle.

4. Procédé (200) selon l'une quelconque des revendications précédentes, le système d'analyse d'imagerie fonctionnelle comportant des moyens de restitution (5) du signal physiologique reconstruit à un utilisateur (6) dudit système, lesdits moyens de restitution (5) coopérant avec l'unité de traitement (4), ledit procédé comportant une étape subséquente (290) pour déclencher une restitution du signal physiologique reconstruit selon un format approprié.

5. Procédé (200) selon l'une quelconque des revendications précédentes, comportant en outre une étape préalable de prétraitement de la donnée expérimentale et/ou du maillage surfacique, ladite étape étant agencée pour corriger et/ou recaler respectivement ladite donnée expérimentale et/ou ledit maillage surfacique.

6. Procédé (200) selon l'une quelconque des revendications précédentes, lorsque le système d'analyse d'imagerie fonctionnelle comporte des moyens de restitution (5) à un utilisateur (6) dudit système, lesdits moyens de restitution (5) coopérant avec l'unité de traitement (4), comportant en outre une étape subséquente pour déclencher la restitution du signal physiologique reconstruit en un ou plusieurs nœuds du maillage pour chaque voxel de la région d'intérêt et générer une image sous la forme d'une carte d'activité fonctionnelle.

7. Unité de traitement (4) d'un système d'analyse d'imagerie fonctionnelle, ladite unité (4) comportant des moyens pour communiquer avec le monde extérieur et des moyens de traitement, coopérant avec des moyens de mémorisation et **caractérisée en ce que** :

- les moyens pour communiquer sont agencés pour recevoir du monde extérieur une donnée expérimentale d'un volume élémentaire d'un organe ;
- les moyens de mémorisation comportent des instructions exécutables ou interprétables par les moyens de traitement dont l'interprétation ou l'exécution desdites instructions par lesdits moyens de traitement provoque la mise en œuvre d'un procédé (200) selon l'une quelconque des revendications 1 à 6.

8. Unité de traitement (4) selon la revendication précédente, pour laquelle les moyens pour communiquer délivrent un signal physiologique reconstruit selon un format approprié à des moyens de restitution (5) aptes à la restituer à un utilisateur (6).

9. Système d'analyse d'imagerie fonctionnelle comportant une unité de traitement (4) selon l'une quelconque des revendications 7 ou 8 et des moyens de restitution (5) aptes à restituer à un utilisateur (6) un signal physiologique selon un procédé (200) conforme à l'une quelconque des revendications 1 à 6 et mis en œuvre par ladite unité de traitement (4).

10. Produit programme d'ordinateur comportant une ou plusieurs instructions interprétables ou exécutables par les moyens de traitement d'une unité de traitement (4) conforme à l'une quelconque des revendications 7 ou 8, ladite unité de traitement (4) comportant en outre des moyens de mémorisation ou coopérant avec de tels moyens de mémorisation, ledit programme étant chargeable dans lesdits moyens de mémorisation, **caractérisé en ce que** l'interprétation ou l'exécution desdites instructions par lesdits moyens de traitement provoque la mise en œuvre d'un procédé (200) selon l'une quelconque des revendications 1 à 6.

**Patentansprüche**

1. Verfahren (200) zur Rekonstruktion eines physiologischen Signals eines dynamischen Arterien-/Gewebe-/Venensystems eines Organs in einem Oberflächenareal, wobei das genannte Verfahren (200) durch Verarbeitungsmittel einer Verarbeitungseinheit (4) eines funktionellen Bildanalysesystems implementiert wird und einen Schritt (270) zum Rekonstruieren des genannten physiologischen Signals auf der Basis von experimentellen Daten einer Region von Interesse, die ein Elementarvolumen - Voxel genannt - des genannten Organs umfasst, und einem Oberflächennetz, das das genannte Oberflächenareal beschreibt, beinhaltet, wobei das genannte Verfahren **dadurch gekennzeichnet ist, dass** der genannte Schritt darin besteht, mit einem Verfahren zur Lösung eines inversen Problems eine A-posteriori-Randverteilung für das genannte physiologische Signal an einem Knoten des genannten Netzes auszuwerten (280) durch:

- Zuweisen (240) einer direkten Wahrscheinlichkeitsverteilung der experimentellen Daten in dem genannten Oberflächenareal unter Kenntnis der Parameter, die in das Problem der Rekonstruktion des physiologischen Signals des dynamischen Arterien-/Gewebe-/Venensystems für das betrachtete Voxel involviert sind;
- gemeinsames Zuweisen (250) einer räumlichen A-priori-Wahrscheinlichkeitsverteilung des genannten physiologischen Signals durch Einführen einer A-priori-Information bezüglich einer Charakteristik der experimentellen Daten und/oder einer A-priori-Information bezüglich einer Eigenschaft des dynamischen Arterien-/Gewebe-/Venensystems;
- gemeinsames Zuweisen (260) einer zeitlichen A-priori-Wahrscheinlichkeitsverteilung des genannten physiologischen Signals durch Einführen einer A-priori-Information bezüglich der Impulsantwort des genannten dynamischen Arterien-/Gewebe-/Venensystems.

2. Verfahren (200) zur Rekonstruktion eines physiologischen Signals eines dynamischen Arterien-/Gewebe-/Venensystems eines Organs in einem Oberflächenareal, wobei das genannte Verfahren (200) durch Verarbeitungsmittel einer Verarbeitungseinheit (4) eines funktionellen Bildanalysesystems implementiert wird und einen Schritt zum Rekonstruieren des physiologischen Signals auf der Basis von experimentellen Daten einer Region von Interesse, die ein Elementarvolumen - Voxel genannt - umfasst, und einem Oberflächennetz, das das genannte Oberflächenareal beschreibt, beinhaltet, wobei das genannte Verfahren **dadurch gekennzeichnet ist, dass** der genannte Schritt darin besteht, mit einem Verfahren zur Lösung eines inversen Problems eine Kostenfunktion für das genannte physiologische Signal an einem Knoten des genannten Netzes auszuwerten (270) durch:

- Zuweisen (240) eines Operators des direkten Modells, der die Verbindung zwischen den experimentellen Daten in dem Elementarvolumen und dem genannten physiologischen Signal in dem genannten Oberflächenareal herstellt, unter Kenntnis der Parameter, die in das Problem der Rekonstruktion des physiologischen Signals des dynamischen Arterien-/Gewebe-/Venensystems für das betrachtete Voxel involviert sind;
- gemeinsames Zuweisen (250) eines räumlichen Regularisierungsoperators durch Einführen einer A-priori-Information bezüglich einer Charakteristik der experimentellen Daten und/oder einer A-priori-Information bezüglich einer Eigenschaft des dynamischen Arterien-/Gewebe-/Venensystems;
- gemeinsames Zuweisen (260) eines zeitlichen Regularisierungsoperators durch Einführen einer A-priori-Information bezüglich der Impulsantwort des genannten dynamischen Arterien-/Gewebe-/Venensystems.

3. Verfahren (200) nach einem der vorherigen Ansprüche, das ferner einen Schritt (230) zum Erzeugen der genannten experimentellen Daten auf der Basis einer Signalerfassung durch funktionelle Bildgebung beinhaltet.

4. Verfahren (200) nach einem der vorherigen Ansprüche, wobei das funktionelle Bildanalysesystem Mittel (5) zum Wiederherstellen des rekonstruierten physiologischen Signals für einen Benutzer (6) des genannten Systems enthält, wobei die genannten Wiederherstellungsmittel (5) mit der Verarbeitungseinheit (4) zusammenwirken, wobei das genannte Verfahren einen nachfolgenden Schritt (290) zum Auslösen einer Wiederherstellung des rekonstruierten physiologischen Signals gemäß einem geeigneten Format beinhaltet.

5. Verfahren (200) nach einem der vorherigen Ansprüche, das ferner einen Vorabschritt des Vorverarbeitens der experimentellen Daten und/oder des Oberflächennetzes beinhaltet, wobei der genannte Schritt zum Korrigieren und/oder Neueinstellen jeweils der genannten experimentellen Daten und/oder des genannten Oberflächennetzes ausgelegt ist.

6. Verfahren (200) nach einem der vorherigen Ansprüche, wenn das funktionelle Bildanalysesystem Mittel (5) zur Wiederherstellung für einen Benutzer (6) des genannten Systems enthält, wobei die genannten Wiederherstellungsmittel (5) mit der Verarbeitungseinheit (4) zusammenwirken, ferner mit einem nachfolgenden Schritt zum Auslösen der Wiederherstellung des an einem oder mehreren Knoten des Netzes rekonstruierten physiologischen Signals für jedes Voxel der Region von Interesse und zum Erzeugen eines Bildes in Form einer funktionellen Aktivitätskarte.

7. Verarbeitungseinheit (4) eines funktionellen Bildanalysesystems, wobei die genannte Einheit (4) Mittel zur Kommunikation mit der Außenwelt und Verarbeitungsmittel umfasst, die mit Speichermitteln zusammenarbeiten, und **dadurch gekennzeichnet, dass**:

- die Kommunikationsmittel zum Empfangen von experimentellen Daten eines Elementarvolumens eines Organs von der Außenwelt ausgelegt sind;
- die Speichermittel Befehle umfassen, die durch die Verarbeitungsmittel ausgeführt oder interpretiert werden können, wobei die Interpretation oder Ausführung der genannten Befehle durch die genannten Verarbeitungsmittel das Implementieren eines Verfahrens (200) nach einem der Ansprüche 1 bis 6 bewirkt.

8. Verarbeitungseinheit (4) nach dem vorherigen Anspruch, für die die Kommunikationsmittel ein gemäß einem geeigneten Format rekonstruiertes physiologisches Signal an Wiederherstellungsmittel (5) liefern, die es für einen Benutzer (6) wiederherstellen können.

9. Funktionelles Bildanalysesystem mit einer Verarbeitungseinheit (4) nach Anspruch 7 oder 8 und Wiederherstellungsmitteln (5), die ein physiologisches Signal für einen Benutzer (6) mit einem von der genannten Verarbeitungseinheit (4) ausgeführten Verfahren (200) gemäß einem der Ansprüche 1 bis 6 wiederherstellen können.

10. Computerprogrammprodukt, das einen oder mehrere Befehle enthält, die durch die Verarbeitungsmittel einer Verarbeitungseinheit (4) gemäß Anspruch 7 oder 8 interpretiert oder ausgeführt werden können, wobei die genannte Verarbeitungseinheit (4) außerdem Speichermittel enthält oder mit solchen Speichermitteln zusammenwirkt, wobei das genannte Programm in die genannten Speichermittel geladen werden kann, **dadurch gekennzeichnet, dass** die Interpretation oder Ausführung der genannten Befehle durch die genannten Verarbeitungsmittel das Implementieren eines Verfahrens (200) nach einem der Ansprüche 1 bis 6 bewirkt.

**Claims**

1. A method (200) for reconstructing a physiological signal of an artery/tissue/vein system of an organ in a surface space, said method (200) being implemented by processing means of a processing unit (4) of a functional imaging analysis system, and comprising a step (270) for reconstructing said physiological signal from an experimental datum of a region of interest comprising an elementary volume-called voxel-of said organ and a surface mesh describing said surface space, said method being **characterized in that** said step consists of evaluating (280), according to a method for solving an inverse problem, an *a posteriori* marginal distribution for said physiological signal in a vertex of said mesh by:

   - assigning (240) a direct probability distribution of the experimental datum in said surface space knowing the parameters involved in the reconstruction problem of the physiological signal of the artery/tissue/vein dynamic system for the voxel in question;
   - jointly assigning (250) an *a priori* spatial probability distribution of said physiological signal by introducing *a priori* information relative to a characteristic of the experimental datum and/or *a priori* information relative to a property of the artery/tissue/vein dynamic system;
   - jointly assigning (260) an *a priori* temporal probability distribution of said physiological signal by introducing *a priori* information relative to the impulse response of said artery/tissue/vein dynamic system.

2. A method (200) for reconstructing a physiological signal of an artery/tissue/vein system of an organ in a surface space, said method (200) being implemented by processing means of a processing unit (4) of a functional imaging analysis system, and comprising a step for reconstructing said physiological signal from an experimental datum of a region of interest comprising an elementary volume-called voxel-and a surface mesh describing said surface space, said method being **characterized in that** said step consists of evaluating (270), according to a method for solving an inverse problem, a cost function for said physiological signal in a vertex of said mesh by:

   - assigning (240) an operator of the direct model establishing the link between the experimental datum in the elementary volume and said physiological signal in said surface space knowing the parameters involved in the problem of the reconstruction of the physiological signal of the artery/tissue/vein dynamic system for the voxel in question;
   - jointly assigning (250) a spatial regularization operator by introducing *a priori* information relative to a characteristic of the experimental datum and/or *a priori* information relative to a property of the artery/tissue/vein dynamic system;
   - jointly assigning (260) a temporal regularization operator by introducing *a priori* information relative to the impulse response of said artery/tissue/vein dynamic system.

3. The method (200) according to any one of the preceding claims, further comprising a step (230) for producing said experimental datum from an acquisition of a signal by functional imaging.

4. The method (200) according to any one of the preceding claims, the functional imaging analysis system comprising output means (5) for the reconstructed physiological signal for a user (6) of said system, said output means (5) cooperating with the processing unit (4), said method comprising a subsequent step (290) for triggering a output of the reconstructed physiological signal in an appropriate format.

5. The method (200) according to any one of the preceding claims, further comprising a prior step for preprocessing of the experimental datum and/or the surface mesh, said step being arranged to correct and/or recalibrate the experimental datum and/or the surface mesh, respectively.

6. The method (200) according to any one of the preceding claims, when the functional imaging analysis system comprises output means (5) for a user (6) of said system, said output means (5) cooperating with the processing unit (4), further comprising a subsequent step for triggering the output of the reconstructed physiological signal in one or several vertices of the mesh for each voxel of the region of interest and generating an image in the form of a functional activity map.

7. A processing unit (4) of a functional imaging analysis system, said unit (4) comprising means for communicating with the outside world and processing means, cooperating with storage means and **characterized in that**:

   - the communication means are arranged to receive, from the outside world, an experimental datum from an elementary volume of an organ,

- the storage means comprise instructions executable or interpretable by the processing means, whereof the interpretation or execution of said instructions by said processing means causes the implementation of a method (200) according to any one of claims 1 to 6.

8. The processing unit (4) according to the preceding claim, for which the communication means deliver a reconstructed physiological signal in an appropriate format to output means (5) suitable for retrieving it for a user (6).

9. A functional imaging analysis system comprising a processing unit (4) according to any one of claims 7 or 8 and output means (5) able to output, for a user (6), a physiological signal using a method (200) according to any one of claims 1 to 6 and implemented by said processing unit (4).

10. A computer program product comprising one or several instructions interpretable or executable by the processing means of a processing unit (4) according to any one of claims 7 or 8, said processing unit (4) further comprising storage means or cooperating with such storage means, said program being loadable in said storage means, **characterized in that** the interpretation or execution of said instructions by said processing means causes the implementation of a method (200) according to any one of claims 1 to 6.

FIG.1

FIG.2

FIG.3

FIG.4

200

210

230

211-n

211

211-y

212

240

241-n

241

241-y

242

250

251-n

251

251-y

252

260

261-n

261

261-y

262

270

280

290

300

**FIG.5**

**FIG.6A**          **FIG.6B**          **FIG.6C**

**FIG.7**

**FIG.8A**

**FIG.8B**

**FIG.8C**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Projection of fMRI data onto the cortical surface using anatomically-informed convolution kernels. **OPERTO G et al.** NEUROIMAGE. ELSEVIER, 01 Janvier 2008, vol. 39, 127-135